# EUROPEAN PATENT SPECIFICATION

(11) **EP 3 289 103 B1**
(45) Date of publication and mention of the grant of the patent: **06.10.2021**
(21) Application number: 16730054.0
(22) Date of filing: 02.05.2016
(51) Int. Cl.: C12Q 1/6827, C12Q 1/6823

(54) **MULTIPLEX INVASIVE CLEAVAGE ASSAYS**
MULTIPLEXE INVASIVE SPALTUNGSTESTS
ESSAIS DE CLIVAGE INVASIF MULTIPLEXE

(30) Priority: 01.05.2015 US 201562156043 P
(43) Date of publication of application: 07.03.2018
(73) Proprietor: Gen-Probe Incorporated, San Diego, CA 92121 (US)
(72) Inventor: PETERSON, Patrick L., San Marcos, California 92069 (US); KING, Joe, Madison, Wisconsin 53705 (US); HALL, Jeff, Waunakee, Wisconsin 53597 (US)
(74) Representative: Script IP Limited
(86) International application number: PCT/US2016/030416
(87) International publication number: WO 2016/179093

(56) References cited:
- US-A1- 2005 074 788
- US-A1- 2008 199 936
- US-A1- 2010 105 575
- US-A1- 2012 122 105
- US-B2- 7 309 573

## Description

### FIELD

The present disclosure relates generally to the field of biotechnology. More particularly, the disclosure relates to multiplex invasive cleavage assays that permit independent detection of a plurality of target nucleic acids with temporal distinction using single channel fluorescence monitoring.

### BACKGROUND

Detection of specific nucleic acid sequences using target amplification and/or hybridization-based signal amplification represents a flexible approach for identifying organisms, single nucleotide polymorphisms, mutations, and the like. Nucleic acid amplification techniques include isothermal amplification (*e.g.,* Transcription Mediated Amplification, or "TMA") reactions, and reactions that involve thermal cycling (*e.g.,* the polymerase chain reaction, or "PCR"). Separate from these are signal amplification reactions that may even bypass the need for increasing the target copy level prior to detection. Invasive cleavage reactions represent exemplary signal amplification reactions.

Some or most of the informative nucleic acid assays, whether hybridization-based or amplification-based assays, rely on "multiplexing," where multiple targets can be detected or quantified in a single reaction mixture. Common multiplex reactions involve first increasing the target copy level of a plurality of nucleic acid analytes that may be present in a test sample. This process, commonly referred to as "multiplex amplification," is often followed by detection of either the aggregated collection of amplified targets, or followed by detection of individuals among the collection of amplified targets using unique probes labeled with different reporter moieties.

Unfortunately, the ability to carry out multiplex nucleic acid analysis is highly constrained. For example, developing oligonucleotide probes and primers that do not participate in undesired side reactions can be complicated. The use of multiple detectable labels can also require different detection "channels" on analytical instruments, which may be limited in the range of detectable wavelengths. In some instances, the use of a single detectable label for detecting more than one target compromises the ability to identify the target responsible for generating the signal.

Some assays designed for multiplex detection of amplified nucleic acids have relied on a single reporter for detection of a plurality of target nucleic acids. For example, U.S. 2012/0003646 A1 disclosed detection of an amplified internal control (IC) and an amplified viral target sequence using different probes that harbored identical acridinium ester labels. Both amplification products were detected simultaneously under a single set of assay conditions, meaning that results were not gathered independently or in a manner that distinguished the origins of the signals. A differential threshold analysis was applied to establish the presence of either IC alone, or the combination of internal control and target as a combination. By this approach the viral analyte could be detected, but individual contributions due to internal control and analyte remained obscure.

In view of the history of multiplex nucleic acid detection, and further in view of the desire for automated systems for conducting multiplex assays, there remains a need that goes beyond the existing alternatives. The present disclosure answers these needs by providing an approach wherein multiple analytes can be detected and distinguished from each other in a procedure that involves monitoring only a single detectable label.

US2005/074788 describes detection of small nucleic acids.

### SUMMARY

The aspects and embodiments of the present invention are set forth in the claims.

Aspects of this disclosure relate to a method of determining which of a plurality of different target nucleic acids are present in a test sample. In one aspect, the method includes the step of, in a reaction mixture containing nucleic acids from the test sample, serially permitting a plurality of secondary reactions of a multiplex invasive cleavage assay to take place, each of the secondary reactions being active under a different temperature condition, and each secondary reaction being specific for only one of the target nucleic acids. The method further includes the step of monitoring the reaction mixture for production of a fluorescent signal under each of the different temperature conditions, each of the different temperature conditions being permissive for only one of the secondary reactions, and each of the secondary reactions producing the same fluorescent signal. Still further, the method includes the step of determining which of the target nucleic acids is present in the test sample based on the fluorescent signal produced in the reaction mixture under each of the different temperature conditions. In a first preferred embodiment, the test sample includes products of a nucleic acid amplification reaction, and each of the plurality of target nucleic acids is a product of the nucleic acid amplification reaction. In a second preferred embodiment, the test sample includes products of a multiplex nucleic acid amplification reaction, and each of the plurality of target nucleic acids is a product of the multiplex nucleic acid amplification reaction. According to a generally preferred embodiment, the reaction mixture includes a thermostable DNA polymerase enzyme. According to another generally preferred embodiment, each of the different temperature conditions differs from the others by 5°C to 30°C. Preferably, each of the different temperature conditions may differ from the others by 5°C to 15°C. According to another generally preferred embodiment, the determining step involves determining which of the target nucleic acids is present in the test sample based on the amount of the fluorescent signal produced in the reaction mixture under each of the different temperature conditions. According to another generally preferred embodiment, the determining step involves determining which of the target nucleic acids is present in the test sample based on the rate of production of the fluorescent signal in the reaction mixture under each of the different temperature conditions.

In another aspect, the disclosed approach relates to a method of determining whether a test sample contains one or both of a first target nucleic acid and a second target nucleic acid. The method begins with the step of preparing a reaction mixture by combining each of: (i) the test sample; (ii) a first set of oligonucleotides for conducting a first invasive cleavage assay specific for detection of the first target nucleic acid, the first invasive cleavage assay including a primary reaction and a secondary reaction, and the first set of oligonucleotides including a first invasive probe specific for the first target nucleic acid, a first primary probe specific for the first target nucleic acid, and a first FRET cassette; (iii) a second set of oligonucleotides for conducting a second invasive cleavage assay specific for detection of the second target nucleic acid, the second invasive cleavage assay including a primary reaction and a secondary reaction, the second set of oligonucleotides including a second invasive probe specific for the second target nucleic acid, a second primary probe specific for the second target nucleic acid, and a second FRET cassette; and (iv) a flap endonuclease (FEN) enzyme. Notably, each of the first and second FRET cassettes includes a donor moiety and an acceptor moiety in energy transfer relationship such that emission from the donor moiety is quenched when the donor moiety and the acceptor moiety are both attached to the same FRET cassette, and the donor moiety of the first FRET cassette is identical to the donor moiety of the second FRET cassette. The method further includes the step of first incubating the reaction mixture for a first period of time at a first temperature, the first temperature being permissive for the primary and secondary reactions of the first invasive cleavage assay, whereby a first signal associated with the donor moiety of the first FRET cassette is generated if the test sample includes the first target nucleic acid. The method further includes the step of first measuring any of the first signal that may have been generated during the first incubating step as an indication of the presence of the first target nucleic acid in the test sample. The method further includes the step of second incubating the reaction mixture for a second period of time at a second temperature, the second temperature being permissive for the secondary reaction of the second invasive cleavage assay, whereby a second signal associated with the donor moiety of the second FRET cassette is generated if the test sample includes the second target nucleic acid. The method further includes the step of second measuring any of the second signal that may have been generated during the second incubating step as an indication of the presence of the second target nucleic acid in the test sample. Finally, there is the step of determining from the first signal measured in the first measuring step whether the sample contains the first target nucleic acid, and from the second signal measured in the second measuring step whether the sample contains the second target nucleic acid. Generally speaking, the secondary reaction of the first invasive cleavage assay does not substantially generate any signal greater than a background signal at the second temperature in the second incubating step, and the secondary reaction of the second invasive cleavage assay does not substantially generate any signal greater than a background signal at the first temperature in the first incubating step. In one example, the primary reaction of the second invasive cleavage assay takes place either at the first temperature in the first incubating step or at the second temperature in the second incubating step. For example, the primary reaction of the second invasive cleavage assay can take place at the first temperature in the first incubating step, and not at the second temperature in the second incubating step. Alternatively, the primary reaction of the second invasive cleavage assay can take place at the second temperature in second incubating step, and not at the first temperature in the first incubating step. In another preferred embodiment, the donor moiety is a fluorophore. In still another preferred embodiment, the first and second temperatures in the incubating steps differ by 5-30°C. More preferably, the first and second temperatures in the incubating steps differ by 5-15°C. Regardless of whether the first and second temperatures in the incubating steps differ by 5-30°C or by 5-15°C, the second measuring step can include measuring over time any of the second signal that may have been generated during the second incubating step, whereby a rate of change of the signal generated in the second incubating step is established. It still a different preferred embodiment, the first measuring step can include identifying the magnitude of the first signal that may have been generated during the first incubating step, and the determining step further includes determining that the first target nucleic acid is present in the sample if the identified magnitude exceeds a magnitude threshold for detecting the first target nucleic acid. In still a different preferred embodiment, the first measuring step can include identifying the magnitude of the first signal that may have been generated during the first incubating step, and the detecting step further includes determining that the first target nucleic acid is not present in the sample if the identified magnitude does not exceed a magnitude threshold for detecting the first target nucleic acid. In still a different preferred embodiment, the second measuring step can include measuring over time any of the second signal that may have been generated during the second incubating step, whereby a rate of change of the signal generated in the second incubating step is established, and the determining step further includes determining that the second target nucleic acid is present in the sample if the rate of change exceeds a velocity threshold for detecting the second target nucleic acid. In still a different preferred embodiment, the second measuring step can include measuring over time any of the second signal that may have been generated during the second incubating step, whereby a rate of change of the signal generated in the second incubating step is established, and the determining step further includes determining that the second target nucleic acid is not present in the sample if the rate of change does not exceed a velocity threshold for detecting the second target nucleic acid. In some embodiments, when the rate of change of the signal generated in the second incubating step is established, the rate of change is a time-dependent rate of change. In some other embodiments, when the rate of change of the signal generated in the second incubating step is established, the rate of change is a cycle-dependent rate of change. In still yet other embodiments, the threshold for detecting the second target can be a threshold velocity. In certain generally preferred embodiments, the first temperature of the first incubating step is greater than the second temperature of the second incubating step. In certain generally preferred embodiments, the threshold for detecting the first target nucleic acid in the determining step is a threshold magnitude. In certain generally preferred embodiments, the first incubating and measuring steps, and the second incubating and measuring steps are repeated in sequence a plurality of times before performing the determining step.

In another aspect, the disclosed approach relates to a computer program product for determining with a multiplex invasive cleavage assay carried out in a reaction mixture whether a test sample contains either a first target nucleic acid or a second target nucleic acid. The computer program product includes non-transitory instructions causing a computer to carry out a number of steps. First, there is the step of collecting a set of test data by monitoring, with a fluorometer that detects emission from a fluorophore in the reaction mixture of the multiplex invasive cleavage assay, fluorescent signals generated by the secondary reaction of a first invasive cleavage assay specific for the first target nucleic acid but not the second target nucleic acid, and the secondary reaction of a second invasive cleavage assay specific for the second target nucleic acid but not the first target nucleic acid. The first invasive cleavage assay uses the fluorophore to indicate the presence of the first target nucleic acid, and the second invasive cleavage assay uses the fluorophore to indicate the presence of the second target nucleic acid. The secondary reaction of the first invasive cleavage assay proceeds at a first temperature that substantially does not permit generation of fluorescent signals by the secondary reaction of the second invasive cleavage assay. As well, the secondary reaction of the second invasive cleavage assay proceeds at a second temperature that substantially does not permit generation of fluorescent signals by the secondary reaction of the first invasive cleavage assay. The set of test data includes: a first background signal measured after the secondary reaction of the first invasive cleavage assay is substantially complete and before the secondary reaction of the second invasive cleavage assay has substantially begun; and at least one additional signal collected after the secondary reaction of the second invasive cleavage assay has begun at the second temperature. Next, there is the step of calculating a velocity indicating how rapidly the at least one additional signal of the set of test data increases above the first background signal. Next, there is the step of comparing the set of test data and the velocity of the calculating step with a set of control data, the set of control data comprising results generated by monitoring with a fluorometer a control reaction mixture that did not include either the first target nucleic acid or the second target nucleic acid. The set of control data includes: a control background signal; at least one additional signal collected after incubation of the control reaction mixture at the second temperature for a period of time; and a calculated velocity indicating how rapidly the at least one additional signal of the set of control data increases above the control background signal. Finally, there is the step of outputting one of the following determinations: (i) the test sample contains the first target nucleic acid if the first background signal is substantially greater than the control background signal; (ii) the test sample does not contain the first target nucleic acid if the first background signal is substantially the same as the control background signal; (iii) the test sample contains the second target nucleic acid if the velocity calculated in the calculating step exceeds the calculated velocity from the set of control data; and (iv) the test sample does not contain the second target nucleic acid if the velocity calculated in the calculating step does not exceed the calculated velocity from the set of control data. In one example, the outputting step involves instructing a display device to produce a tangible record of the determination. When this is the case, the calculating step can involve calculating the slope of a line. Alternatively, the display device can be a printer, and the tangible record can be a printed record. Generally speaking, the collecting step can involve receiving numerical values for the set of test data, and then sorting the numerical values into an electronic spreadsheet. According to another example, the calculated velocity is in units of RFU/minute. According to yet another example, the calculating step involves calculating the slope of a line. Preferably, the velocity calculated in the calculating step is in units of RFU/minute. According to still yet another example, the fluorometer used in the comparing step is the same fluorometer used in the collecting step. According to still yet another example, the reaction mixture is contained in a reaction vessel positioned in an instrument that amplifies and detects nucleic acids, and the collecting step is performed at only one temperature. According to still yet another example, the computer program product is loaded into the computer, and the collecting step does not involve collecting the set of test data by monitoring as a function of time fluorescent signals generated at more than one temperature.

In another aspect, the disclosed approach relates to a computer program product that includes non-transitory instructions causing a computer to carry out particular steps. First, there is the step of collecting a first set of fluorescent signal data by monitoring emission from a fluorophore in the secondary reaction of a first invasive cleavage assay that detects a first target nucleic acid in a test sample, the first invasive cleavage assay being conducted in a reaction mixture at a first temperature that substantially does not permit generation of fluorescent signals by other secondary reactions using the fluorophore in the reaction mixture. The first set of fluorescent signal data includes: a first background signal determined after any preceding secondary reaction generating increased fluorescence from the fluorophore in the reaction mixture was concluded, but before the secondary reaction of the first invasive cleavage assay has substantially begun; and at least one additional signal collected after the secondary reaction of the first invasive cleavage assay has begun Next, there is the step of collecting a second set of fluorescent signal data by monitoring emission from the fluorophore in the secondary reaction of a second invasive cleavage assay that detects a second target nucleic acid in the test sample, the second invasive cleavage assay being conducted in the reaction mixture at a second temperature that substantially does not permit generation of fluorescent signals by other secondary reactions using the fluorophore in the reaction mixture. The second set of fluorescent signal data includes: a second background signal determined after any preceding secondary reaction generating increased fluorescence from the fluorophore in the reaction mixture was concluded, but before the secondary reaction of the second invasive cleavage assay has substantially begun; and at least one additional signal collected after the secondary reaction of the second invasive cleavage assay has begun. Next, there is the step of preparing each of a first modified data set and a second modified data set from the respective first and second sets of fluorescent signal data. The modified first data set is prepared by subtracting the first background signal from each of the at least one additional signal of the first set of fluorescent signal data. The modified first data set indicates fluorescent signal due to the presence of the first nucleic acid in the test sample. The modified second data set is prepared by subtracting the second background signal from each of the at least one additional signal of the second set of fluorescent signal data. The modified first data set indicates fluorescent signal due to the presence of the first nucleic acid in the test sample. Finally, there is the step of outputting a result indicating the presence or absence of each of the first target nucleic acid and the second target nucleic acid based on the modified first and second data sets, respectively. In one example, the two collecting steps involve receiving numerical values for the respective first and second sets of fluorescent signal data, and then sorting the numerical values into an electronic spreadsheet. In another example, the first temperature is higher than the second temperature. In still another example, the first temperature is lower than the second temperature. In yet still another example, the outputting step involves instructing a display device to produce a tangible record of the result. For example, the display device can be a printer, and the tangible record can be a record printed on paper. In yet still another example, the computer is a component of an apparatus that cycles the reaction mixture from the first temperature to the second temperature a plurality of times. In yet still another example, the computer program product is loaded into the computer, and the computer is a component of an apparatus that cycles the reaction mixture from the first temperature to the second temperature a plurality of times.

In another aspect, the disclosed approach relates to a system for independently determining the presence or absence of each of a first and a second analyte target nucleic acid using a multiplex invasive cleavage assay carried out in a reaction mixture. Among the components of the system is a temperature-controlled incubator that receives a reaction vessel containing the reaction mixture. The temperature-controlled incubator is configured to incubate the reaction mixture at a first temperature for a first period of time to permit a first secondary reaction of the multiplex invasive cleavage assay to take place, and thereafter to incubate the reaction mixture at a second temperature for a second period of time to permit a second secondary reaction of the multiplex invasive cleavage assay to take place. Another component of the system is a fluorometer that measures in a single detection channel any fluorescent signal of a predetermined wavelength range that may be generated in the reaction mixture at each of the first and second temperatures during the respective first and second periods of time. Another component of the system is a computer in communication with the fluorometer. The computer is programmed to associate measured fluorescent signal increases generated during each of the time periods with the presence of a different one of the analyte target nucleic acids. The computer associates any measured fluorescent signal increase during the first period of time with the presence of the first analyte target nucleic acid, and not with the presence of the second analyte target nucleic acid. As well, the computer associates any measured fluorescent signal increase during the second period of time with the presence of the second analyte target nucleic acid, and not with the presence of the first analyte target nucleic acid. Another component of the system is an output device, in communication with the computer, that generates a tangible record indicating the presence or absence of each of the first and second analyte target nucleic acids. In an example, the first and second temperatures differ by 5°C to 30°C. According to one example, the temperature-controlled incubator includes a thermal cycler. According to another example, the temperature-controlled incubator includes one or more temperature-controlled chambers. For example, each of the one or more temperature-controlled chambers can maintain a constant temperature. According to another example, the output device is a printer, and the tangible record generated by the output device is printed on paper. According to another example, the temperature-controlled incubator includes a plurality of temperature-controlled chambers, and the system further includes a controller that directs changes in the position of the reaction vessel within the plurality of temperature-controlled chambers. According to another example, the reaction vessel containing the reaction mixture is any of a multiwell plate, an individual tube, or a multi-tube unit comprising a linear array of tubes. According to another example, the single detection channel measures the fluorescence emission of fluorescein but not the fluorescence emission of ROX. According to another example, the single detection channel measures the fluorescence emission of ROX but not the fluorescence emission of fluorescein. According to another example, the temperature-controlled incubator is configured by software instructions specific for the multiplex invasive cleavage assay to incubate the reaction mixture at the first temperature for the first period of time, and to incubate the reaction mixture at the second temperature for the second period of time. According to another example, the first temperature is higher than the second temperature. According to another example, the first temperature is lower than the second temperature. According to another example, the fluorometer includes a plurality of detection channels, and only one of the detection channels is used for independently detecting the first and second analyte target nucleic acids.

In other aspects, this disclosure relates to a kit for performing a temperature-dependent multiplex invasive cleavage assay. The kit includes first and second FRET cassettes in one or more receptacles, with each of the first and second FRET cassettes having a different 5' flap hybridizing sequence, and each of the first and second FRET cassettes including a donor moiety and an acceptor moiety, where the donor moieties of the first and second FRET cassettes are equivalent donor moieties. In certain preferred kits, the first and second FRET cassettes are contained in a single receptacle. In other preferred kits, the donor moieties of the first and second FRET cassettes are identical. For example, the donor moieties of the first and second FRET cassettes can be identical fluorophores. Optionally, the acceptor moieties of the first and second FRET cassettes are identical. When this is the case, the acceptor moieties of the first and second FRET cassettes can be identical quencher moieties. Optionally, kits can include in one or more additional receptacles, first and second primary probes, with each of the first and second primary probes including a 3' target hybridizing sequence and a 5' flap sequence, where the 3' target hybridizing sequence of the first primary probe is complementary to a first region of a first target nucleic acid, and where the 3' target hybridizing sequence of the second primary probe is complementary to a first region of a second target nucleic acid, where the 3' target hybridizing sequences of the first and second primary probes are different from each other, where the 5' flap sequences of the first and second primary probes are complementary to the 5' flap hybridizing sequences of the first and second FRET cassettes, respectively, where the 5' flap sequences of the first and second primary probes are different from each other, and where the melting temperature of a first hybrid formed between the 5' flap hybridizing sequence of the first FRET cassette and a cleaved form of the 5' flap sequence of the first primary probe differs by at least 5°C from the melting temperature of a second hybrid formed between the 5' flap hybridizing sequence of the second FRET cassette and a cleaved form of the 5' flap sequence of the second primary probe. In examples, the melting temperatures of the first and second hybrids differ by 5°C to 30°C. More preferably, the melting temperatures of the first and second hybrids differ by 5°C to 15°C. Optionally, the first and second FRET cassettes and the first and second primary probes are contained in a single receptacle. Alternatively, the kit may further include in the one or more additional receptacles, first and second invasive probes, where the first invasive probe is complementary to a second region of the first target nucleic acid, the first and second regions of the first target nucleic acid being situated adjacent to each other, and where the second invasive probe is complementary to a second region of the second target nucleic acid, with the first and second regions of the second target nucleic acid being situated adjacent to each other. In an example, the first and second FRET cassettes and the first and second invasive probes are contained in a single receptacle. Generally, the kit may further include in a receptacle that does not contain the FRET cassettes or the primary probe, a flap endonuclease (FEN) enzyme. Generally, the donor moieties of the first and second FRET cassettes exhibit emission spectra having measurable intensities within a range of from 500 nm to 750 nm. For example, the donor moieties of the first and second FRET cassettes exhibit emission spectra having measurable intensities within a range of from 510 nm to 530 nm, 560 nm to 580 nm, 610 nm to 650 nm, 675 nm to 690 nm, or 705 nm to 730 nm. Generally, the donor moieties of the first and second FRET cassettes exhibit emission spectra having peak emission wavelengths that differ by 0 nm to 15 nm. Preferably, the donor moieties of the first and second FRET cassettes exhibit emission spectra having peak emission wavelengths that differ by 0 nm to 10 nm. More preferably, the donor moieties of the first and second FRET cassettes exhibit emission spectra having peak emission wavelengths that differ by 0 nm to 5 nm. In an example, each of the first and second FRET cassettes is free in solution.

In another aspect, the disclosed approach generally relates to a composition of matter and related reaction mixtures. In an example, the composition includes a mixture of first and second FRET cassettes, with each of the first and second FRET cassettes having a different 5' flap-hybridizing sequence, and each of the first and second FRET cassettes including a donor moiety and an acceptor moiety, where the donor moieties of the first and second FRET cassettes are equivalent donor moieties. In an example, the donor moieties of the first and second FRET cassettes are identical. For example, the donor moieties of the first and second FRET cassettes can be identical fluorophores. Optionally, the acceptor moieties of the first and second FRET cassettes are identical. When this is the case, the acceptor moieties of the first and second FRET cassettes can be identical quencher moieties. Generally, the melting temperature of a first hybrid formed between the 5' flap hybridizing sequence of the first FRET cassette and a fully complementary first 5' flap sequence differs by at least 5°C from the melting temperature of a second hybrid formed between the 5' flap hybridizing sequence of the second FRET cassette and a fully complementary second 5' flap sequence, and where the first and second 5' flap sequences are different from each other. In an example, the melting temperatures of the first and second hybrids differ by 5°C to 30°C. More preferably, the melting temperatures of the first and second hybrids differ by 5°C to 15°C. Generally speaking, the donor moieties of the first and second FRET cassettes preferably exhibit emission spectra having measurable intensities within a range of from 500 nm to 750 nm. For example, the donor moieties of the first and second FRET cassettes can exhibit emission spectra having measurable intensities within a range of from 510 nm to 530 nm, 560 nm to 580 nm, 610 nm to 650 nm, 675 nm to 690 nm, or 705 nm to 730 nm. Preferably, the donor moieties of the first and second FRET cassettes exhibit emission spectra having peak emission wavelengths that differ by 0 nm to 15 nm. More preferably, the donor moieties of the first and second FRET cassettes exhibit emission spectra having peak emission wavelengths that differ by 0 nm to 10 nm. Still more preferably, the donor moieties of the first and second FRET cassettes exhibit emission spectra having peak emission wavelengths that differ by 0 nm to 5 nm. Generally speaking, compositions, kits and reaction mixtures may further include a flap endonuclease (FEN) enzyme. As well, each of the first and second FRET cassettes can be free in solution, and the 5' flap hybridizing sequences of the first and second FRET cassettes can each be in a single-stranded state. Other examples of the disclosure relate to reaction mixtures for detecting two or more target nucleic acids in a multiplex invasive cleavage assay. In an example, a reaction mixture includes first and second FRET cassettes, with each of the first and second FRET cassettes having a different 5' flap hybridizing sequence, where each of the first and second FRET cassettes includes a donor moiety and an acceptor moiety and where the donor moieties of the first and second FRET cassettes are equivalent donor moieties; where the reaction mixture further includes first and second primary probes, where each of the first and second primary probes includes a 3' target hybridizing sequence and a 5' flap sequence and where the 3' target hybridizing sequence of the first primary probe is complementary to a first region of a first target nucleic acid, where the 3' target hybridizing sequence of the second primary probe is complementary to a first region of a second target nucleic acid, where the 3' target hybridizing sequences of the first and second primary probes are different from each other, where the 3' target hybridizing sequences of the first and second primary probes are complementary to the 5' flap hybridizing sequences of the first and second FRET cassettes, respectively, and where the 5' flap sequences of the first and second primary probes are different from each other; and where the reaction mixture further includes first and second invasive probes, where the first invasive probe is complementary to a second region of the first target nucleic acid, where the first and second regions of the first target nucleic acid are situated adjacent to each other, and where the second invasive probe is complementary to a second region of the second target nucleic acid, where the first and second regions of the second target nucleic acid are situated adjacent to each other. In another example of a reaction mixture according to this disclosure, the melting temperature of a first hybrid formed between the 5' flap hybridizing sequence of the first FRET cassette and a cleaved form of the 5' flap sequence of the first primary probe differs by at least 5°C from the melting temperature of a second hybrid formed between the 5' flap hybridizing sequence of the second FRET cassette and a cleaved form of the 5' flap sequence of the second primary probe.

### Definitions

As used herein, the term "sample" refers to a specimen that may contain an analyte of interest (*e.g.*, microbe, virus, nucleic acid such as a gene, or components thereof, which includes nucleic acid sequences in or derived from an analyte). Samples may be from any source, such as biological specimens or environmental sources. Biological specimens include any tissue or material derived from a living or dead organism that may contain an analyte or nucleic acid in or derived from an analyte. Examples of biological samples include vaginal swab samples, respiratory tissue, exudates (*e.g*., bronchoalveolar lavage), biopsy, sputum, peripheral blood, plasma, serum, lymph node, gastrointestinal tissue, feces, urine, or other fluids, tissues or materials. Examples of environmental samples include water, ice, soil, slurries, debris, biofilms, airborne particles, and aerosols. Samples may be processed specimens or materials, such as obtained from treating a sample by using filtration, centrifugation, sedimentation, or adherence to a medium, such as matrix or support. Other processing of samples may include treatments to physically or mechanically disrupt tissue, cellular aggregates, or cells to release intracellular components that include nucleic acids into a solution which may contain other components, such as enzymes, buffers, salts, detergents and the like. Samples being tested for the presence of an analyte may sometimes be referred to as "test samples."

As used herein, an "invasive cleavage assay" is a procedure that detects or quantifies a target nucleic acid by enzymatic cleavage of two different invasive cleavage structures. Reagents for an invasive cleavage assay include: a structure-specific 5' nuclease; and three oligonucleotides (an "invasive probe," a "primary probe," and a "FRET cassette"). The invasive cleavage assay combines two invasive signal amplification reactions (*i.e*., a "primary reaction" and a "secondary reaction") in series in a single reaction mixture.

References to "first" and "second" invasive cleavage assays simply provides identifiers for distinguishing one invasive cleavage assay from another, without necessarily indicating one precedes the other.

A "reaction mixture" is a combination of reagents (*e.g.*, oligonucleotides, target nucleic acids, enzymes, *etc.*) in a single reaction vessel.

As used herein, a "multiplex" assay is a type of assay that measures multiple analytes (two or more) in a single run of the assay. It is distinguished from procedures that measure one analyte per reaction mixture. A multiplex invasive cleavage assay is carried out by combining into a single reaction vessel the reagents for two or more different invasive cleavage assays. Preferably, the same species of fluorescent reporter is detected in each of the assays of the multiplex.

As used herein, the term "invasive cleavage structure" (or simply "cleavage structure") refers to a structure comprising: (1) a target nucleic acid, (2) an upstream nucleic acid (*e.g.,* an invasive probe oligonucleotide), and (3) a downstream nucleic acid (*e.g.,* a primary probe oligonucleotide), where the upstream and downstream nucleic acids anneal to contiguous regions of the target nucleic acid, and where an overlap forms between the a 3' portion of the upstream nucleic acid and duplex formed between the downstream nucleic acid and the target nucleic acid. An overlap occurs where one or more bases from the upstream and downstream nucleic acids occupy the same position with respect to a target nucleic acid base, whether or not the overlapping base(s) of the upstream nucleic acid are complementary with the target nucleic acid, and whether or not those bases are natural bases or non-natural bases. In some examples, the 3' portion of the upstream nucleic acid that overlaps with the downstream duplex is a non-base chemical moiety such as an aromatic ring structure, as disclosed, for example, in U.S. Patent No. 6,090,543. In some examples, one or more of the nucleic acids may be attached to each other, for example through a covalent linkage such as nucleic acid stem-loop, or through a non-nucleic acid chemical linkage *(e.g.,* a multi-carbon chain). An invasive cleavage structure also is created when a cleaved 5' flap hybridizes to a FRET cassette *(i.e.,* when the "target nucleic acid" and the "downstream nucleic acid" are covalently linked in a stem-loop configuration). The "target nucleic acid" sequence of a FRET cassette that hybridizes to a cleaved 5' flap can be referred to as a "5' flap-hybridizing sequence."

The term "complementary," as used herein, refers to nucleobase sequences that are capable of forming a double-stranded, hydrogen bonded region. The nucleobase sequences may be "perfectly complementary" (i.e., each nucleobase in one sequence is capable of pairing with a corresponding nucleobase in a second sequence) or they may be "partially complementary" (i.e., at least one nucleobase in one sequence is incapable of hydrogen bonding with a corresponding nucleobase in a second sequence). The nucleobase sequences may be in the same or different polynucleotides.

The term "hybrid," as used herein, refers to a nucleic acid structure comprising a double-stranded, hydrogen-bonded region. Such structures may be fully or partially double-stranded and include RNA:RNA, RNA:DNA and DNA:DNA duplex molecules and analogs thereof. By way of example, a "hybrid" includes a cleaved 5' flap sequence hybridized to a complementary 5' flap hybridizing sequence of a FRET cassette.

The term "cleaved form," as used herein, refers to a portion of a polynucleotide that has been cleaved from the remainder of the polynucleotide by the action of one or more nucleases. By way of example, a 5' flap sequence is in a "cleaved form" when a primary probe has been cleaved by an endonuclease (e.g., a FEN enzyme), thereby separating the 5' flap sequence from the target hybridizing sequence of the primary probe.

As used herein, the term "flap endonuclease" or "FEN" (*e.g.,* "FEN enzyme") refers to a class of nucleolytic enzymes that act as structure-specific endonucleases on DNA structures with a duplex containing a single-stranded 5' overhang, or flap, on one of the strands that is displaced by another strand of nucleic acid, such that there are overlapping nucleotides at the junction between the single and double-stranded DNA. FEN enzymes catalyze hydrolytic cleavage of the phosphodiester bond 3' adjacent to the junction of single and double stranded DNA, releasing the overhang, or "flap" (*see* Trends Biochem. Sci. 23:331-336 (1998) and Annu. Rev. Biochem. 73: 589-615 (2004)). FEN enzymes may be individual enzymes, multi-subunit enzymes, or may exist as an activity of another enzyme or protein complex, such as a DNA polymerase. A flap endonuclease may be thermostable. Examples of FEN enzymes useful in the methods disclosed herein are described in U.S. Patent Nos. 5,614,402; 5,795,763; 6,090,606; and in published PCT applications identified by WO 98/23774; WO 02/070755; WO 01/90337; and WO 03/073067. Particular examples of commercially available FEN enzymes include the Cleavase® enzymes (Hologic, Inc.).

The term "single-stranded state," as used herein with reference to a polynucleotide, refers to a region of the polynucleotide that is available for base pairing. In the case of a single-stranded polynucleotide having self-complementary regions, the term "single-stranded state" is a reference to a region of the self-complementary polynucleotide that is available for base pairing. By way of example, the 5' flap hybridizing sequence of a FRET cassette is in a single-stranded state prior to hybridizing to a complementary 5' flap sequence.

As used herein, the term "probe" refers to an oligonucleotide that interacts with a target nucleic acid to form a detectable complex. Examples include invasive probes and primary probes. An "invasive probe" refers to an oligonucleotide that hybridizes to a target nucleic acid at a location near the region of hybridization between a primary probe and the target nucleic acid, wherein the invasive probe oligonucleotide comprises a portion (*e.g.,* a chemical moiety, or nucleotide, whether complementary to that target or not) that overlaps with the region of hybridization between the primary probe oligonucleotide and the target nucleic acid. The "primary probe" includes a target-specific region that hybridizes to the target nucleic acid, and further includes a "5' flap" region that is not complementary to the target nucleic acid.

As used herein, the term "primary reaction" refers to enzymatic cleavage of a primary probe, whereby a cleaved 5' flap is generated. The sequence of the cleaved 5' flap will be the 5' flap sequence of the primary probe (*i.e.,* the sequence not complementary to the target nucleic acid), and one base at its 3' terminus from the target-specific region (*i.e.,* the sequence complementary to the target nucleic acid) of the primary probe.

As used herein, the term "secondary reaction" refers to enzymatic cleavage of a FRET cassette (following hybridization of a cleaved 5' flap) to generate a detectable signal.

As used herein, a reaction is "active" when reaction products are generated. For example, a secondary reaction is active when a reaction mixture containing necessary components (*e.g.*, including a FRET cassette, a cleaved 5' flap specific for the FRET cassette, and a FEN enzyme) are incubated at a temperature that permits cycling hybridization of the cleaved 5' flap to FRET cassettes in the reaction mixture to result in cleavage of the FRET cassettes and separation of donor and acceptor moieties.

As used herein, "temperature conditions" used for conducting a primary or secondary reaction refer to the temperature, or range of temperatures, that permit a reaction to take place. Different temperature profiles of different reactions mean that temperature conditions that allow one reaction to take place may not allow a different reaction to take place.

As used herein, "optimal" (and grammatical variants thereof) reaction conditions refer to the most favorable reaction conditions for promoting or allowing a reaction to take place. For example, an optimal temperature for performing a secondary reaction would be the temperature at which a FRET cassette was cleaved most efficiently in a reaction mixture (*e.g.*, corresponding to the peak on a plot of signal-to-noise ratio as a function of reaction temperature). Similarly, an optimal temperature range would be a range of most favorable temperature conditions for promoting or allowing a reaction to take place. In certain examples, preferred optimal temperature ranges may include the optimal temperature plus-or-minus 5°C, more preferably plus-or-minus 3°C, still more preferably plus-or-minus 2°C, and yet still more preferably plus-or-minus 1°C.

As used herein, the term "donor" refers to a moiety (*e.g.*, a fluorophore) that absorbs at a first wavelength and emits at a second, longer wavelength. The term "acceptor" refers to a moiety such as a fluorophore, chromophore, or quencher and that is able to absorb some or most of the emitted energy from the donor when it is near the donor group (*e.g.,* between 1-100 nm). An acceptor may have an absorption spectrum that overlaps the donor's emission spectrum Generally, if the acceptor is a fluorophore, it then re-emits at a third, still longer wavelength; if it is a chromophore or quencher, it releases the energy absorbed from the donor without emitting a photon. In some examples, alteration in energy levels of donor and/or acceptor moieties are detected (*e.g.*, via measuring energy transfer, for example by detecting light emission) between or from donors and/or acceptor moieties). In some examples, the emission spectrum of an acceptor moiety is distinct from the emission spectrum of a donor moiety such that emissions (*e.g.*, of light and/or energy) from the moieties can be distinguished (*e.g.*, spectrally resolved) from each other.

As used herein, "attached" (*e.g.*, two things are "attached") means chemically bonded together. For example a fluorophore moiety is "attached" to a FRET cassette when it is chemically bonded to the structure of the FRET cassette.

As used herein, the term "FRET cassette" refers to an oligonucleotide, preferably a hairpin structure, that includes a donor moiety and a nearby acceptor moiety, where attachment of the donor and acceptor moieties to the same FRET cassette substantially suppresses (*e.g.*, quenches) a detectable energy emission (*e.g.*, a fluorescent emission). The nucleotide base sequence of the portion of the FRET cassette that hybridizes to a complementary cleaved 5' flap to form an invasive cleavage structure (*i.e*., the substrate for a FEN enzyme) is referred to herein as the 5' flap-hybridizing sequence. In this respect, two 5' flap-hybridizing sequences are said to be "different" from each other when their sequences are not the same over their full lengths. Cleavage of the FRET cassette by a FEN enzyme in a secondary reaction separates the donor and acceptor moieties with the result of relieving the suppression and permitting generation of a signal. In some examples, the donor and acceptor moieties interact by fluorescence resonance energy transfer (*e.g.*, "FRET"). In other examples, the donor and acceptor of the FRET cassette interact by a non-FRET mechanism.

As used herein, an "interactive" label pair refers to a donor moiety and an acceptor moiety being attached to the same FRET cassette, and being in energy transfer relationship (*i.e.,* whether by a FRET or a non-FRET mechanism) with each other. A signal (*e.g.,* a fluorescent signal) can be generated when the donor and acceptor moieties are separated, for example by cleavage of the FRET cassette in a secondary reaction. Different FRET cassettes that specifically hybridize to different cleaved 5' flaps can each include the same interactive label pair.

As used herein, "equivalent" (*e.g.*, in the context of "equivalent donor-acceptor pairs," or "equivalent donor" or "equivalent fluorophore" moieties) means that excitation and emission spectra of detectable chemical species are sufficiently similar or overlapping by wavelength range as to permit detection of fluorescent emission wavelengths in the same channel of an instrument used for monitoring signals.

As used herein, the term "spectral overlap" refers to two or more light spectra with at least one common wavelength.

As used herein, emission from a donor moiety (*e.g.*, a fluorophore) is "quenched" when the emission of a photon from the donor is prevented because an acceptor moiety (*e.g.,* a quencher) is sufficiently close. For example, emission from a donor moiety is quenched when the donor moiety and the acceptor moiety are both attached to the same FRET cassette.

As used herein, the term "hybridize" or "hybridization" is used in reference to the pairing of complementary nucleic acids. Hybridization and the strength of hybridization (*i.e*., the strength of the association between the nucleic acids) is impacted by such factors as the degree of complementary between the nucleic acids, stringency of the conditions involved, the Tm of the formed hybrid, and the G:C ratio within the nucleic acids.

As used herein, the term "Tm" is used in reference to the "melting temperature." The melting temperature is the temperature at which a population of double-stranded nucleic acid molecules becomes half dissociated into single strands. The equation for calculating the Tm of nucleic acids is well known in the art.

As used herein, "specific" means pertaining to only one (or to only a particularly indicated group), such as having a particular effect on only one (or on only a particularly indicated group), or affecting only one (or only a particularly indicated group) in a particular way. For example, a cleaved 5' flap specific for a FRET cassette will be able to hybridize to that FRET cassette, form an invasive cleavage structure, and promote a cleavage reaction, but will not be able to hybridize to a different FRET cassette (*e.g.*, a FRET cassette having a different 5' flap-hybridizing sequence) to promote a cleavage reaction.

As used herein, the term "specifically hybridizes" means that under given hybridization conditions a probe or primer detectably hybridizes substantially only to the target sequence in a sample comprising the target sequence (*i.e*., there is little or no detectable hybridization to non-target sequences).

The term "thermostable" when used in reference to an enzyme, such as a FEN enzyme, indicates that the enzyme is functional or active (*i.e*., can perform catalysis) at an elevated temperature (*i.e*., at about 55°C or higher). In some examples, the enzyme is functional or active at an elevated temperature of 65°C or higher (*e.g.,* 75°C, 85°C, 95°C, *etc.*).

As used herein, the terms "target nucleic acid" and "target sequence" refer to a nucleic acid that is to be detected or analyzed. Thus, the "target" is sought to be distinguished from other nucleic acids or nucleic acid sequences. For example, when used in reference to an amplification reaction, these terms may refer to the nucleic acid or portion of nucleic acid that will be amplified by the reaction, while when used in reference to a polymorphism, they may refer to the portion of a nucleic acid containing a suspected polymorphism. When used in reference to an invasive cleavage reaction, these terms refer to a nucleic acid molecule containing a sequence that has at least partial complementarity with at least a first nucleic acid molecule (*e.g.* primary probe oligonucleotide) and also have at least partial complementarity with a second nucleic acid molecule (*e.g.* invasive probe oligonucleotide). Target nucleic acids from an organism may comprise any nucleic acid species, including but not limited to genomic DNAs and RNAs, messenger RNAs, structural RNAs, ribosomal and tRNAs, and small RNAs such as snRNAs, siRNAs and microRNAs (miRNAs).

As used herein, the term "amplified" refers to an increase in the abundance of a molecule, moiety or effect. A target nucleic acid may be amplified, for example by *in vitro* replication such as by PCR.

As used herein, the term "amplification method" as used in reference to nucleic acid amplification means a process of specifically amplifying the abundance of a nucleic acid of interest. Some amplification methods (*e.g.*, polymerase chain reaction, or PCR) comprise iterative cycles of thermal denaturation, oligonucleotide primer annealing to template molecules, and nucleic acid polymerase extension of the annealed primers. Conditions and times necessary for each of these steps are well known in the art. Some amplification methods are conducted at a single temperature and are deemed "isothermal." Accumulation of the products of amplification may be exponential or linear. Some amplification methods ("target amplification" methods) amplify the abundance of a target sequence by copying it many times (*e.g.*, PCR, NASBA, TMA, strand displacement amplification, ligase chain reaction, LAMP, ICAN, RPA, SPA, HAD, *etc.),* while some amplification methods amplify the abundance of a nucleic acid species that may or may not contain the target sequence, but the amplification of which indicates the presence of a particular target sequence in the reaction (*e.g.*, rolling circle amplification, RAM amplification). The latter methods are sometimes referred to as "signal amplification" methods. Some signal amplification methods may increase the abundance of a species of nucleic acid by converting a starting nucleic acid, for example by cleaving the starting nucleic acid to form cleavage products, or by extending it by, for example, polymerization or ligation. A target amplification method may be applied to a signal molecule (*e.g.*, PCR may be used to produce more copies of the product of a ligation, cleavage, or non-target copying reaction), or vice versa.

As used herein, the terms "polymerase chain reaction" and "PCR" refer to an enzymatic reaction in which a segment of DNA is replicated from a target nucleic acid *in vitro.* The reaction generally involves extension of a primer on each strand of a target nucleic acid with a template dependent DNA polymerase to produce a complementary copy of a portion of that strand. The chain reaction comprises iterative cycles of denaturation of the DNA strands, for example by heating, followed by cooling to allow primer annealing and extension, resulting in an exponential accumulation of copies of the region of the target nucleic acid that is flanked by and that includes the primer binding sites. When an RNA target nucleic acid is amplified by PCR, it is generally converted to a DNA copy strand with an enzyme capable of reverse transcription. Exemplary enzymes include MMLV reverse transcriptase, AMV reverse transcriptase, as well as other enzymes that will be familiar to those having an ordinary level of skill in the art.

The term "oligonucleotide" as used herein is defined as a molecule comprising two or more nucleotides (*e.g.*, deoxyribonucleotides or ribonucleotides), preferably at least 5 nucleotides, more preferably at least about 10-15 nucleotides and more preferably at least about 15 to 30 nucleotides, or longer (*e.g.,* oligonucleotides are typically less than 200 residues long (*e.g.,* between 15 and 100 nucleotides), however, as used herein, the term is also intended to encompass longer polynucleotide chains). The exact size will depend on many factors, which in turn depend on the ultimate function or use of the oligonucleotide. Oligonucleotides are often referred to by their length. For example a 24 residue oligonucleotide is referred to as a "24-mer." Oligonucleotides can form secondary and tertiary structures by self-hybridizing or by hybridizing to other polynucleotides. Such structures can include, but are not limited to, duplexes, hairpins, cruciforms, bends, and triplexes. Oligonucleotides may be generated in any manner, including chemical synthesis, DNA replication, reverse transcription, PCR, or a combination thereof. In some examples, oligonucleotides that form invasive cleavage structures are generated in a reaction (*e.g.*, by extension of a primer in an enzymatic extension reaction). As used herein, the terms "oligonucleotide" and "polynucleotide" may be used interchangeably and may comprise non-naturally occurring monomers, or portions thereof. More particularly, oligonucleotides may include, for example, linear or circular oligomers of natural and/or modified monomers or linkages, including deoxyribonucleosides, ribonucleosides, substituted and alpha-anomeric forms thereof, peptide nucleic acids (PNA), locked nucleic acids (LNA), 2'-O-methyl modifications, phosphorothioate, methylphosphonate, spacers and the like.

As used herein, a "signal" is a detectable quantity or impulse of energy, such as electromagnetic energy (*e.g.*, light). Emission of light from an appropriately stimulated fluorophore is an example of a fluorescent signal. In some examples, "signal" refers to the aggregated energy detected in a single channel of a detection instrument (*e.g.*, a fluorometer).

As used herein, a "background" signal is the signal (*e.g.,* a fluorescent signal) generated under conditions that do not permit a target nucleic acid-specific reaction to take place. For example, signal generated in a secondary reaction that includes a FRET cassette and FEN enzyme, but not a cleaved 5' flap would produce a background signal. In some instances, a background signal is measured in a "negative control" trail that omits the target nucleic acid.

As used herein a "channel" of an energy sensor device, such as a device equipped with an optical energy sensor, refers to a pre-defined band of wavelengths that can be detected or quantified to the exclusion of other bands of wavelengths. For example, one detection channel of a fluorometer might be capable of detecting light energy emitted by one or more fluorescent labels over a range of wavelengths as a single event. Light emitted as the result of fluorescence can be quantified as relative fluorescence units (RFU) at a given wavelength, or over a band of wavelengths. Examples of common fluorescence detection channels include those that detect fluorescence emission wavelengths in the ranges of from about 510-530 nm (*e.g.*, common for a "FAM" detection channel), from about 560-580 nm (*e.g.,* common for a "HEX" detection channel), from about 610-650 nm (*e.g.,* common for a "Texas Red" detection channel), from about 675-690 nm (*e.g.*, common for a "Cy5" detection channel), and from about 705-730 nm (*e.g*., common for a "Quasar 705" detection channel). Cy5 and Alexa Fluor® 647 are examples of two different fluorophores that may be detected in the Cy5 channel of a fluorometer.

As used herein, the term "allele" refers to a variant form of a given sequence (e.g., including but not limited to, genes containing one or more single nucleotide polymorphisms or "SNPs"). A large number of genes are present in multiple allelic forms in a population. A diploid organism carrying two different alleles of a gene is said to be heterozygous for that gene, whereas a homozygote carries two copies of the same allele.

The term "wild-type" (also "WT" herein) refers to a gene or gene product that has the characteristics of that gene or gene product when isolated from a naturally occurring source. A wild-type gene is that which is most frequently observed in a population and is thus arbitrarily designated the "normal" or "wild-type" form of the gene. In contrast, the terms "modified," "mutant" (also "Mut" herein), and "variant" refer to a gene or gene product that displays modifications in sequence and or functional properties (*i.e.,* altered characteristics) when compared to the wild-type gene or gene product.

As used herein, a "threshold" or "threshold cutoff' refers to a quantitative limit used for interpreting experimental results, where results above and below the cutoff lead to opposite conclusions. For example, a measured signal falling below a cutoff may indicate the absence of a particular target, but a measured signal that exceeds the same cutoff may indicate the presence of that target. By convention, a result that meets a cutoff (*i.e*., has exactly the cutoff value) is given the same interpretation as a result that exceeds the cutoff.

As used herein, a "threshold cycle number" refers to indicia of amplification that measure the time or cycle number when a real-time run curve signal crosses an arbitrary value or threshold. "TTime" and "Ct" determinations are examples of threshold-based indicia of amplification. Other methods involve performing a derivative analysis of the real-time run curve. For the purpose of this disclosure, TArc and OTArc also can be used to determine when a real-time run curve signal crosses an arbitrary value (*e.g.*, corresponding to a maximum or minimum angle in curvature, respectively). Methods of TTime determination are disclosed in U.S. 8,615,368; methods of Ct determination are disclosed in EP 0640828 B1; derivative-based methods are disclosed in U.S. 6,303,305; and methods of TArc and OTArc determination are disclosed in U.S. 7,739,054. Those having an ordinary level of skill in the art will be aware of variations that also can be used for determining threshold cycle numbers.

As used herein, an "internal calibrator" nucleic acid is a nucleic acid that can be amplified in an *in vitro* nucleic acid amplification reaction, and that is distinguishable from an analyte nucleic acid (e*.g.,* the target nucleic acid from a test sample) that is coamplified in the same reaction. "Internal" means that the calibrator nucleic acid is amplified and detected within the same reaction mixture as the analyte target nucleic acid, or fragment thereof. In some examples, the internal calibrator nucleic acid is amplified by the same primer(s) used for amplifying the analyte nucleic acid that is to be quantified. In other examples, different primers are used for this purpose. Generally speaking, the analyte nucleic acid and the internal calibrator nucleic acid differ by at least one nucleotide position that can be discriminated by an invasive cleavage reaction. This allows multiplexed invasive cleavage assays to detect amplified internal calibrator and analyte target nucleic acids independently.

As used herein, a "calibration standard" is a composition that includes a known or predetermined amount of an internal calibrator nucleic acid.

As used herein, a "reaction vessel" or "reaction receptacle" is a container for containing a reaction mixture. Examples include individual wells of a multiwell plate, and plastic tubes (*e.g.*, including individual tubes within a formed linear array of a multi-tube unit, *etc.*). However, it is to be understood that any suitable container may be used for containing the reaction mixture.

As used herein, the phrase "temporal" separation or isolation of invasive cleavage reactions refers to a plurality of reactions, all being detected with the aid of a single fluorophore and/or detection channel in the same reaction mixture, where at least the secondary reactions take place at different times (*i.e*., secondary reactions do not occur simultaneously). For example, temperature changes can be used for temporally isolating secondary reactions of multiplexed invasive cleavage assays from each other.

As used herein, "serial" (and grammatical variants thereof) refers to a collection of events or steps occurring in a series rather than simultaneously. For example, serially performing a plurality of reactions means that the reactions are carried out in a sequence (one after the other), and not at the same time. Likewise, serially permitting a plurality of reactions to occur means that the reactions are permitted to occur, for example by providing appropriate temperature conditions, in a sequence (one after the other), and not at the same time.

As used herein, "permitting" a reaction to take place means that reagents and conditions are provided by reaction mixture to test for the presence of a particular nucleic acid (*e.g.,* a target DNA, or a cleaved 5' flap), which may or may not be present in the reaction mixture. For example, "permitting" a primary reaction of an invasive cleavage assay to take place means that a reaction mixture includes an invasive probe, a primary probe that includes a 5' flap sequence, and a FEN enzyme under appropriate buffer and temperature conditions to allow cleavage of the primary probe and release of a cleaved 5' flap if a target DNA also is available in the reaction mixture to participate in the primary reaction. Similarly, "permitting" a secondary reaction of an invasive cleavage assay to take place means that a reaction mixture includes a FRET cassette and a FEN enzyme under appropriate buffer and temperature conditions to allow cleavage of the FRET cassette if a cleaved 5' flap specific for the FRET cassette also is available in the reaction mixture to participate in the secondary reaction. Still further, temperature conditions "permitting" (or that "permit" or are "permissive" for) a reaction to take place are temperature conditions that are conducive for conducting or allowing the reaction to proceed.

As used herein, "velocity" of a secondary invasive cleavage reaction refers to the rate of change of a fluorescent signal. Rate, for example, may be measured as a function of time or cycle number.

The term "free in solution," as used herein, refers to a molecule (e.g., polynucleotide) that is neither fixed nor immobilized on a solid support within or containing a liquid medium. By way of example, FRET cassettes are "free in solution" if they are freely dispersed in a liquid medium and not bound to a solid support, such as silica beads, magnetically-responsive particles or microtiter plates.

By "kit" is meant a packaged combination of materials intended for use in conjunction with each other. Kits useful in accordance with the disclosed techniques may include one or more vessels or tubes containing various reagents. Kits further may include instructions, or other information in a "tangible" form (*e.g.*, printed information, electronically recorded on a computer-readable medium, or otherwise recorded on a machine-readable medium such as a bar code).

### BRIEF DESCRIPTION OF THE DRAWINGS

The following figures form part of the present specification and are included to further demonstrate certain aspects and embodiments of the present disclosure. The disclosure may be better understood by reference to one or more of these figures in combination with the description of specific embodiments presented herein.

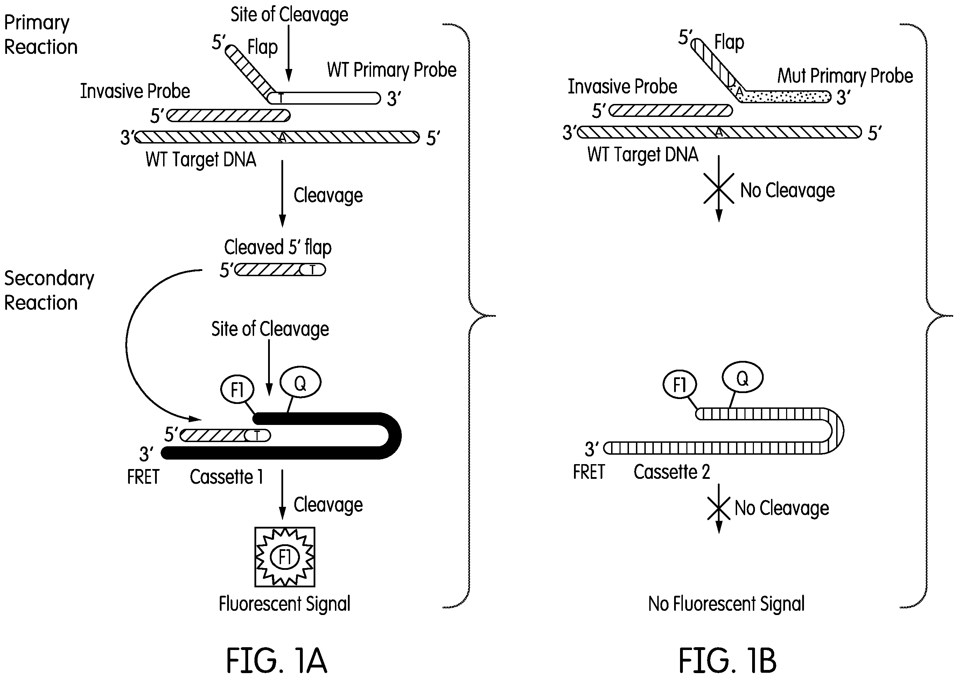

FIGS. 1A-AB include an overview of oligonucleotide components of an invasive cleavage assay in accordance with the disclosure. Different oligonucleotide components participate in primary and secondary reactions of the assay. Wild-type (WT) and mutant (Mut) sequences are indicated. FIG. 1A shows invasive cleavage structures for each of the primary and secondary reactions. The upper invasive cleavage structure includes wild-type target DNA hybridized to an invasive probe and a primary probe, where the two probes are specific for the wild-type target DNA. The lower invasive cleavage structure includes the cleaved 5' flap from the wild-type primary probe, and the FRET1 cassette. The 5' flap-hybridizing sequence of the FRET1 cassette is specific for the cleaved 5' flap from the wild-type primary probe. FIG. 1B shows how the mutant primary probe is not cleaved following interaction with the wild-type target DNA.

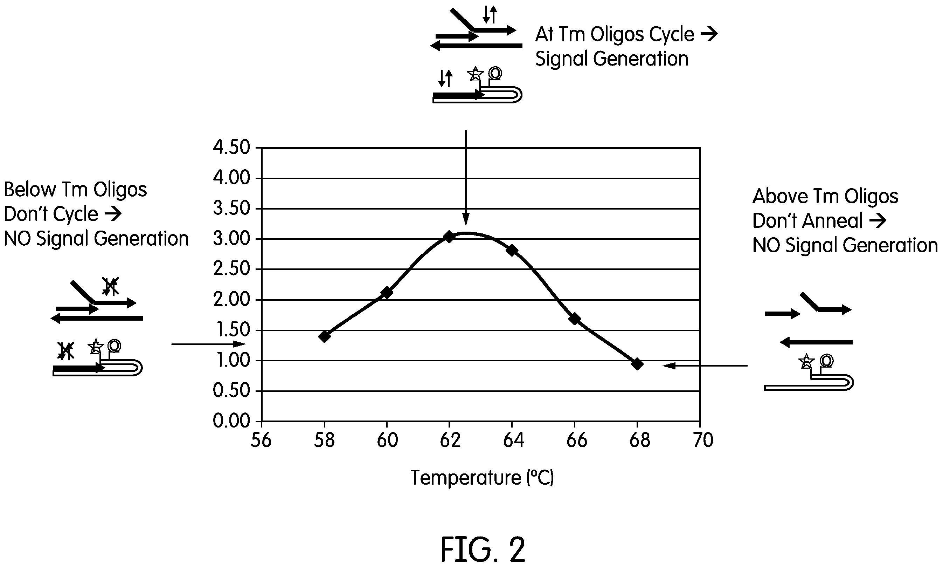

FIG. 2 shows a typical signal profile for an invasive cleavage reaction as a function of reaction temperature (x-axis). The y-axis presents the signal-to-noise ratio for each of the tested reaction conditions. A value of 1.00 indicates substantially no reaction above background. Cycling status of oligonucleotide reagents of the invasive cleavage assay at different points along the curve is depicted by upward/downward arrows.

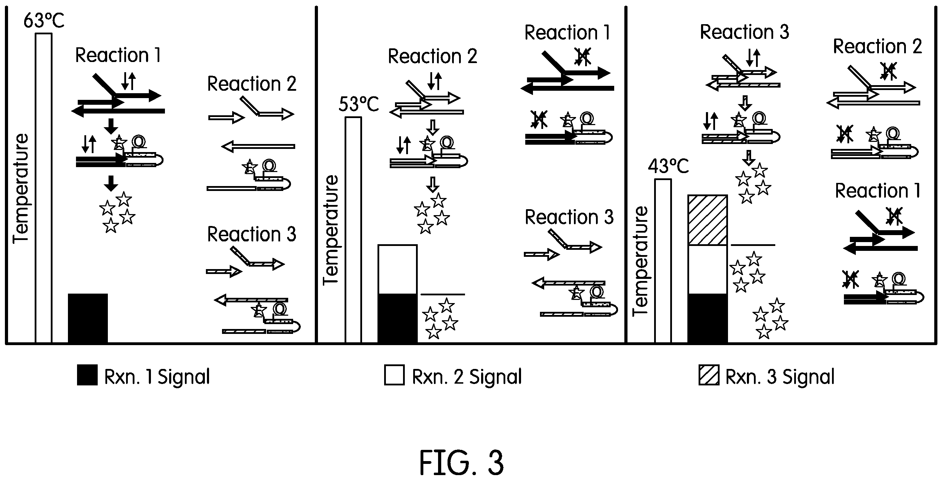

FIG. 3 is a schematic diagram showing the activity of each of three multiplexed invasive cleavage assays under three different temperature conditions.

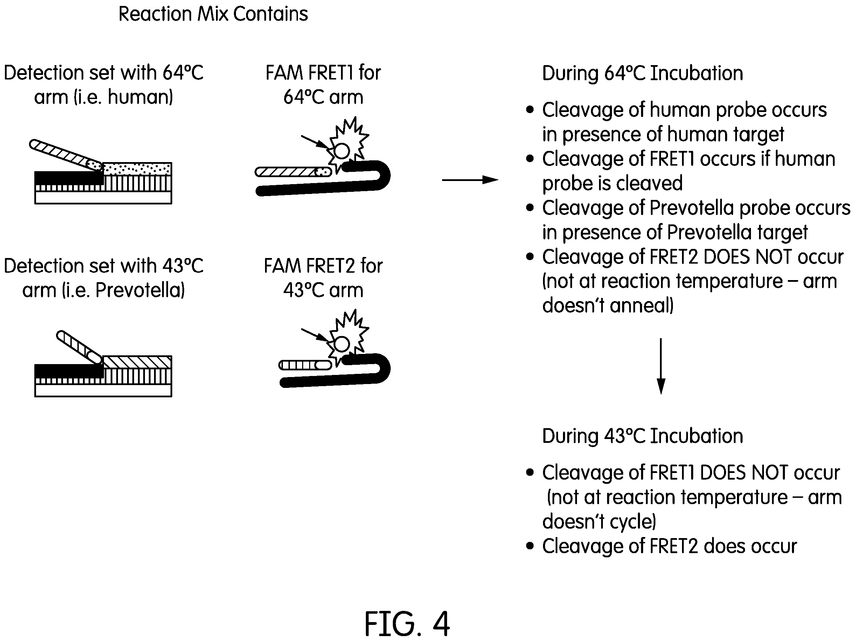

FIG. 4 is a diagram illustrating two different invasive cleavage assays, each of the assays operating at the same optimal temperature for the primary reaction, but operating at a different optimal temperature for the secondary reactions. In the upper assay, both primary and secondary reactions operate at 64°C. In the lower assay, the primary reaction operates at 64°C, but the secondary reaction operates at 43°C. At 64°C the lower secondary reaction does not substantially occur, while at 43°C the upper secondary reaction does not substantially occur. The left portion of the diagram illustrates the cleaved structures resulting from the primary reaction.

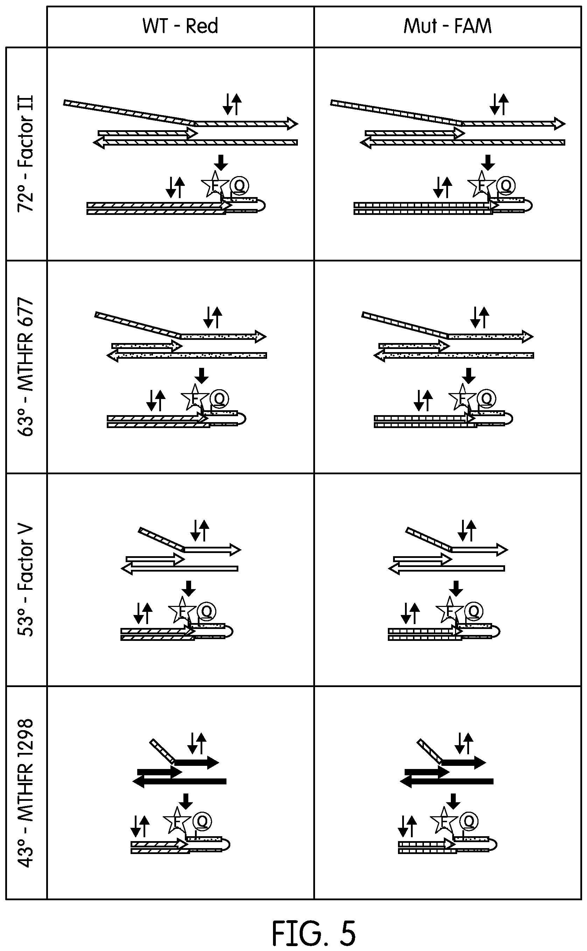

FIG. 5 is a schematic illustration showing oligonucleotides for detecting four wild-type target nucleic acids using FRET cassettes harboring a first fluorophore ("Red"), and reagents for detecting four mutant target nucleic acids using FRET cassettes harboring a second fluorophore ("FAM"). Paired sets of upward/downward arrows indicate oligonucleotides that cycle by hybridization/dissociation at the temperature optima shown at the left of the figure. The 3' ends of target nucleic acids, invasive probes, primary probes, and cleaved 5' flaps interacting with FRET cassettes are indicated by arrowheads. Lengths of the 5' flaps (and cleaved 5' flaps) affect the Tm for interaction with FRET cassettes. Those having an ordinary level of skill in the art will understand that G-C content can be used to alter Tm (*see* below).

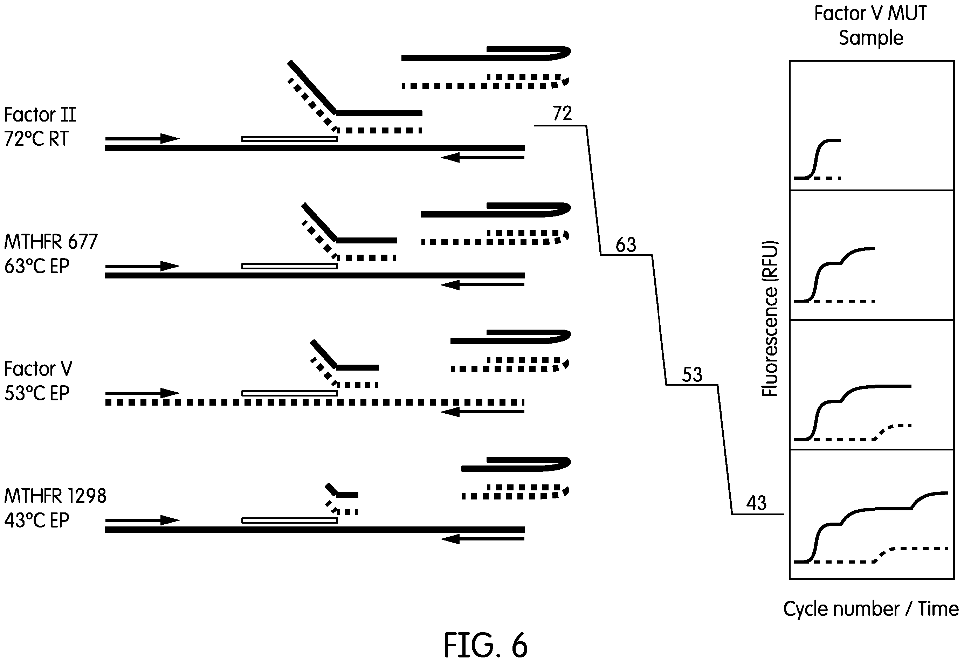

FIG. 6 is a schematic illustration showing four different target nucleic acids, oligonucleotide reagents that can be used in a multiplex invasive cleavage assay, stepped temperature optima for the different individual invasive cleavage assays within the multiplex, and example results (fluorescence as a function of PCR cycle number/read time increment). Four target nucleic acids (*i.e*., wild-type Factor II, wild-type MTHFR 667, mutant Factor V, and wild-type MTHFR 1298) and their associated temperature optima are shown at the left. Primers for amplifying the four target nucleic acids are indicated by lines with arrowheads. Invasive probes are shown hybridized to their respective target nucleic acids. Adjacent to each invasive probe is shown a pair of primary probes (one each being specific for the wild-type or mutant target nucleic acid). Wild-type sequences are indicated by solid lines; and mutant sequences are indicated by broken lines. Differences in 5' flap sequences relate to different Tms. Pairs of FRET cassettes (one for each different 5' flap) also are illustrated. The right side of the figure shows schematic results demonstrating how overall fluorescence is cumulative during the stepped temperature profile.

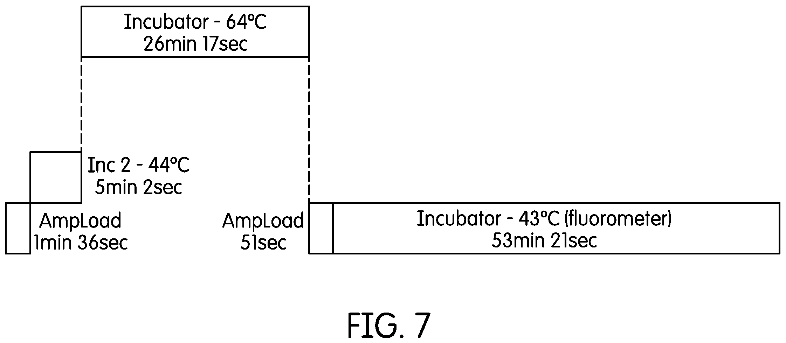

FIG. 7 is a diagrammatic workflow representation showing steps used for conducting reverse transcription (*i.e.,* 44°C), primary invasive cleavage reactions (*i.e.,* 64°C), and secondary invasive cleavage reactions (*i.e.,* being carried out at 64°C for the human-specific assay, and at 43°C for the Prevotella-specific assay). Accumulation of fluorescent label released during the secondary reaction was monitored as a function of time.

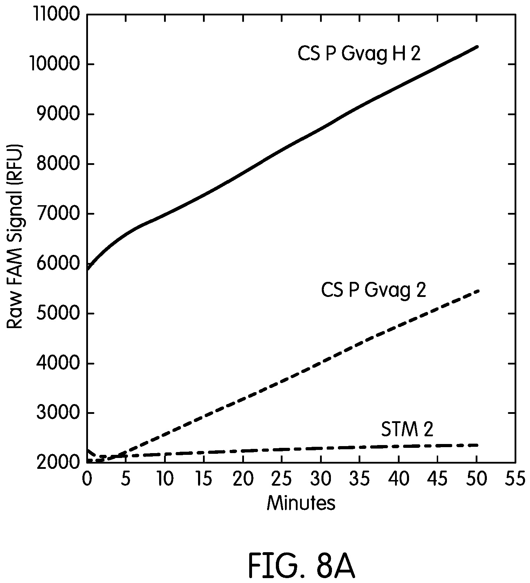

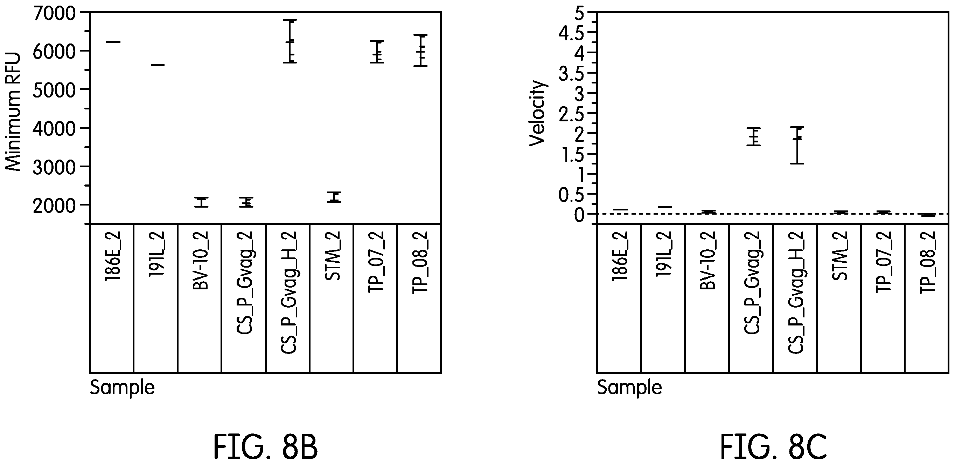

FIGS. 8A-8C are a collection of graphical plots for results obtained in a duplex invasive cleavage assay. Primary and secondary invasive cleavage reactions of the first invasive cleavage assay (*i.e*., for detecting a human target sequence) took place at 64°C. The primary invasive cleavage reaction of the second invasive cleavage assay (*i.e*., for detecting a ribosomal target sequence from bacteria in the genus Prevotella) also took place at 64°C. The secondary invasive cleavage reaction of the second invasive cleavage assay took place at 43°C. Accumulation of fluorescent signal as a function of time is shown only during the portion of the reaction conducted at 43°C. The graph in FIG. 8A shows results for: (a) a negative control sample that did not contain human or bacterial target sequences (lower line); (b) a sample containing the Prevotella target sequence, but not the human target sequence (middle line); and (c) a sample containing both the Prevotella target sequence and the human target sequence (upper line). FIG. 8B shows a plot of the minimum fluorescent signal (measured in RFU) produced by fluoroscein, and corresponding to the y-intercept of the plot shown in FIG. 8A. FIG. 8C shows a plot for velocity of fluorescence accumulation measured for fluorescein during the 43°C incubation.

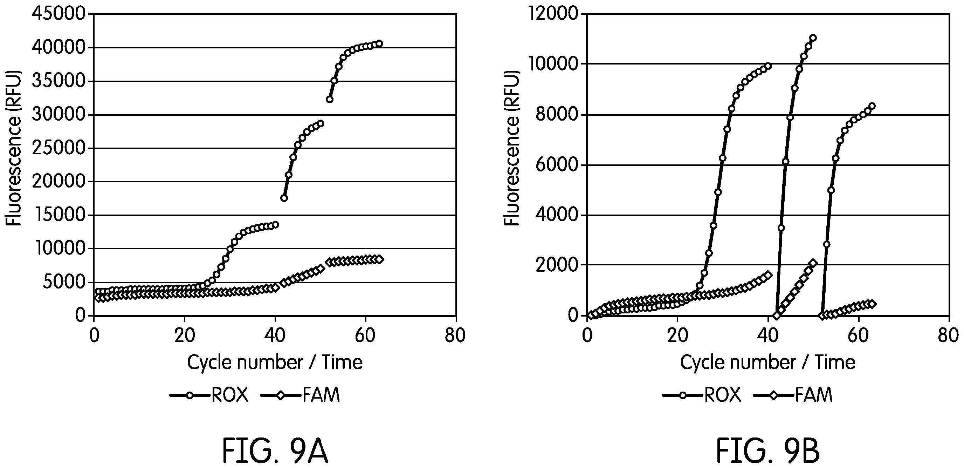

FIGS. 9A-9B are graphs showing fluorescence readings taken during real-time and endpoint formatted segments of a multiplex invasive cleavage assay for detecting nucleic acids encoding proteins of the human blood clotting cascade. Both graphs present fluorescence (RFU) as a function of PCR cycle number and read time. Signals measured in the ROX channel (open circles) were used for detecting wild-type Factor II and Factor V, and mutant MTHFR 1298. Signals measured in the FAM channel (open diamonds) were used for detecting mutant Factor II and Factor V, and wild-type MTHFR 1298. FIG. 9A shows raw fluorescence readings taken during real-time monitoring of the two Factor II invasive cleavage assays in the reaction mixture, as well as readings taken during endpoint formatted invasive cleavage assays for detecting Factor V and MTHFR 1298. FIG. 9B presents data from FIG. 9A following background subtraction to highlight activity for detection of each of the different target nucleic acids in the multiplex assay.

### DETAILED DESCRIPTION

Disclosed herein are compositions, methods, reaction mixtures, kits, computer program products, and systems for performing invasive cleavage assays in a multiplex format. Reaction products indicating the presence of individuals among a plurality of target nucleic acids are detected using a single (*i.e.,* shared) detection channel of an automated system that includes a fluorometer, where fluorescent emissions arising from different invasive cleavage assays preferably result from the same fluorophore species. Generally speaking, the disclosed approach relies on temporal separation of reaction product formation in a multiplex reaction mixture. Temporal separation results from the use of oligonucleotides that permit selective activation of one invasive cleavage reaction (*e.g.*, one secondary reaction) in a predetermined temperature range, while at the same time other invasive cleavage reactions (*e.g.*, a different secondary reaction) mediated by a different set of oligonucleotides in the same reaction mixture are suppressed. Simply stated, temporal separation of secondary reactions provides that the secondary reactions are not actively synthesizing reaction products simultaneously. The temporal separation resulting from controlled temperature shifts is shown herein to be so effective that multiplexing of two, more preferably three, and still more preferably four distinguishable invasive cleavage reactions is now possible. In accordance with the disclosed techniques, multiple invasive cleavage reactions, each generating signals indicating the presence of a different target nucleic acid, can be monitored using only a single fluorophore and detection channel without compromising the ability to discern which of the different targets is present in a test sample.

The disclosed technique does not rely on spatial separation of invasive cleavage reactions to achieve the desired results. There is no requirement for immobilization of any oligonucleotide (*e.g.*, to a bead or planar surface), and so the disclosed technique differs significantly from approaches that may have been employed previously using microarrays. Indeed, the disclosed technique preferably employs oligonucleotide reagents free in solution (*i.e.,* not immobilized), and allows for multiple invasive cleavage reactions to be conducted and distinguished in a single reaction mixture.

As well, the present technique differs significantly from melting curve analyses, where thermal melting of an oligonucleotide hybridized to a target nucleic acid is monitored as a function of changing temperature. Signal indicating the presence of a particular target nucleic acid in accordance with the disclosed technique is produced and gathered at a constant temperature. Signal indicating the presence of a different target nucleic acid is produced and gathered at a different temperature following a shift and hold in the reaction temperature. Simply stated, the present technique does not identify or quantify a target nucleic acid by monitoring the strength or avidity of hybridization as a function of temperature, and does not involve reduction of a signal (*e.g.,* a fluorescent signal) once it is produced. Insofar as gathering accumulated signal is concerned, the present technique would be incompatible with techniques making target identity assignments using melting curve analysis. In addition, hybridization strength is impacted by natural variation which can be informative regarding the existence of sequence variation (*e.g.*, SNPs), but not the location of SNPs. The disclosed technique has the practical advantage of being able to detect a plurality of SNPs using a single detection channel.

### Invasive Cleavage Assays

Oligonucleotide components of an invasive cleavage assay in accordance with the present disclosure are exemplified by the diagrams in FIGS. 1A-1B. The assay provides means for forming an invasive cleavage structure that requires the presence of a target nucleic acid. The assay further involves cleaving the invasive cleavage structure to release distinctive cleavage products. A FEN enzyme, for example, is used to cleave the target-dependent invasive cleavage structure, thereby resulting in cleavage products that indicate the presence of specific target nucleic acid sequences in the sample. When two oligonucleotides hybridize to a target nucleic acid strand such that they form an overlapping invasive cleavage structure, as described below, invasive cleavage can occur by well-known mechanisms. Through the interaction of a cleavage agent *(e.g.,* FEN enzyme) and the upstream oligonucleotide *(i.e.,* the invasive probe), the cleavage agent can be made to cleave the downstream oligonucleotide (*i.e*., the primary probe) at an internal site such that a distinctive fragment is produced. The fragment, sometimes referred to as a "cleaved 5' flap," can then participate as an invasive probe in a subsequent reaction that generates a detectable signal (*e.g.,* a fluorescent signal). Such examples are described in U.S. Pat. Nos. 5,846,717, 5,985,557, 5,994,069, 6,001,567, and 6,090,543, WO 97/27214, WO 98/42873, Nat. Biotech., 17:292 (1999), and PNAS, 97:8272 (2000). Indeed, the INVADER assay disclosed in these references is highly preferred for use as the invasive cleavage assay in the techniques disclosed herein. More specifically, a plurality of INVADER reactions, when combined in a single reaction mixture, and when using a common fluorophore for labeling the FRET cassette(s), are highly preferred for the multiplex applications disclosed herein.

The disclosed multiplex invasive cleavage assay can be used for detecting specific DNA and RNA sequences by using structure-specific enzymes (*e.g.* FEN enzymes) to cleave a complex formed by the hybridization of overlapping oligonucleotide probes. The reaction temperature (*e.g.*, approximating or corresponding to the Tm of the primary probe) enables multiple copies of a primary probe to be cleaved for each target nucleic acid at a constant temperature (*i.e.,* without temperature cycling). These cleaved 5' flaps then direct cleavage of a labeled FRET cassette. The FRET cassette oligonucleotide can be 5'-end labeled with a fluorescent dye that is quenched by an internal dye or quencher moiety. Upon cleavage, the unquenched fluorescent label which is released from the FRET cassette may be detected using standard fluorescence monitoring techniques (*e.g*., a fluorescence plate reader).

The disclosed multiplex invasive cleavage assay can be used for detecting specific target sequences in unamplified, as well as amplified DNA, including genomic DNA, or cDNA synthesized by reverse transcribing RNA. In the examplesshown schematically in FIG. 1A, the assay uses two cascading steps (a primary reaction and a secondary reaction) to generate a target-specific fluorescent signal. For convenience, the alleles in the following discussion are described as wild-type (WT) and mutant (Mut), even though this terminology does not apply to all genetic variations or target sequences. In the primary reaction (FIG. 1A, upper portion), the WT primary probe and the invasive probe hybridize in tandem to the target nucleic acid to form an overlapping structure. An unpaired "flap" is included on the 5' end of the WT primary probe. A structure-specific enzyme (*e.g.* the Cleavase® enzymes available from Hologic, Inc.) recognizes the overlap and cleaves off the unpaired 5' flap and cleavage base. In the secondary reaction (FIG. 1A, lower portion), this cleaved product serves as an invasive probe on the WT FRET cassette to again create a structure recognized by the structure-specific enzyme. When the two dyes on a single FRET cassette are separated by cleavage (indicated by an arrow in FIG. 1A), a detectable fluorescent signal above background fluorescence is produced. Consequently, cleavage of this second invasive cleavage structure results in an increase in fluorescence, indicating the presence of the WT allele (or mutant allele if the assay is configured for the mutant allele to generate the detectable signal).

If the primary probe oligonucleotide and the target nucleotide sequence do not hybridize perfectly at the cleavage site (*e.g.,* as with the Mut primary probe and the WT target shown in FIG. 1B), the overlapped structure does not form and cleavage is suppressed. The structure-specific enzyme (*e.g.*, Cleavase® enzyme available from Hologic, Inc.) used in the procedure cleaves overlapped structures more efficiently than non-overlapping structures, thereby facilitating discrimination of the alleles.

The primary reaction is directed against the target nucleic acid that is being detected. The target nucleic acid is the limiting component in the first invasive cleavage, since the invasive probe and the primary probe are supplied in molar excess. In the second invasive cleavage reaction, it is the released flap that is limiting. When these two cleavage reactions are performed sequentially, the fluorescence signal from the composite reaction accumulates linearly with respect to the target nucleic acid amount.

The disclosed approach may be used to determine the quantity of one or more target nucleic acids present in a reaction using a single (*i.e.,* shared) detection channel. Based on the aforementioned limiting factors of the first invasive cleavage reaction (*e.g.*, target) and the second invasive cleavage reaction (*e.g.*, released flap) it is possible to quantify the level of each target nucleic acid in a given reaction. The two invasive cleavage reactions for a given target nucleic acid may be simultaneous or temporally separated while the second invasive cleavage reactions for other target nucleic acids being detected must be temporally distinct from one another for quantitation to be possible. Example calculations for determining starting target nucleic acid concentration are described in PNAS, 97:8272 (2000).

In certain examples, the target nucleic acid sequence is reverse transcribed, or even amplified prior to detection (*e.g*. such that synthetic nucleic acid is generated). In some examples, the target nucleic acid comprises genomic DNA. In other examples, the target nucleic acid comprises synthetic DNA or RNA. In some examples, synthetic DNA within a sample is created using a purified polymerase. In some examples, creation of synthetic DNA using a purified polymerase comprises the use of PCR. In other examples, creation of synthetic DNA using a purified DNA polymerase, suitable for use with the methods of the present disclosure, comprises use of rolling circle amplification, (*e.g.,* as in U.S. Pat. Nos. 6,210,884, 6,183,960 and 6,235,502. In other examples, creation of synthetic DNA comprises copying genomic DNA by priming from a plurality of sites on a genomic DNA sample. In some examples, priming from a plurality of sites on a genomic DNA sample comprises using short (*e.g.*, fewer than about 8 nucleotides) oligonucleotide primers. In other examples, priming from a plurality of sites on a genomic DNA comprises extension of 3' ends in nicked, double-stranded genomic DNA (*i.e.,* where a 3' hydroxyl group has been made available for extension by breakage or cleavage of one strand of a double stranded region of DNA). Some examples of making synthetic DNA using a purified polymerase on nicked genomic DNAs, suitable for use with the methods and compositions of the present disclosure, are provided in U.S. Pat. Nos. 6,117,634 and 6,197,557, and in published PCT application WO 98/39485.

The present disclosure further provides assays in which the target nucleic acid is reused or recycled during multiple rounds of hybridization with oligonucleotide probes and cleavage of the probes without the need to use temperature cycling (*i.e.,* for periodic denaturation of target nucleic acid strands) or nucleic acid synthesis (*i.e*., for the polymerization-based displacement of target or probe nucleic acid strands). When a cleavage reaction is run under conditions in which the probes are continuously replaced on the target strand (*e.g*. through probe-probe displacement or through an equilibrium between probe/target association and disassociation, or through a combination comprising these mechanisms, (*see* J. Mol. Biol. 97: 511-520 (2000)), multiple probes can hybridize to the same target, allowing multiple cleavages, and the generation of multiple cleavage products.

### Useful Fluorophores and Quenchers

While multiplexed invasive cleavage assays in accordance with the disclosure will employ only a single detection channel for detecting fluorescent signals generated by multiplexed invasive cleavage assays, and preferably will use the same fluorophore for signal generation, the identity of the fluorophore can be variable. Suitable fluorophores for use in the FRET cassettes of the disclosed technique include, but are not limited to: fluorescein, rhodamine, Redmond Red dye, Yakima Yellow dye, hexachloro-fluorescein, TAMRA dye, ROX dye, Cy3, Cy3.5, Cy5, Cy5.5, and Cy7, 4,4-difluoro-5,7-diphenyl-4-bora-3a,4a-diaza- -s-indacene-3-propionic acid, 4,4-difluoro-5,p-methoxyphenyl-4-bora-3a,4a-- diaza-s-indacene-3-propionic acid, 4,4-difluoro-5-styryl-4-bora-3a,4-adiaz- a-S-indacene-propionic acid, 6-carboxy-X-rhodamine, N,N,N',N'-tetramethyl-6-carboxyrhodamine, Texas Red, eosin, fluorescein, 4,4-difluoro-5,7-diphenyl-4-bora-3a,4a-diaza-s-indacene-3-propionic acid, 4,4-difluoro-5,p-ethoxyphenyl-4-bora-3a,4a-diaza-s-indacene 3-propionic acid and 4,4-difluoro-5-styryl-4-bora-3a,4a-diaza-S-indacene-propionic acid, 6-carboxyfluorescein (6-FAM), 2',4',1,4,-tetrachlorofluorescein (TET), 21,4',51,7',1,4-hexachlorofluorescein (HEX), 2',7'-dimethoxy-4',5'-dichloro-6-carboxyrhodamine (JOE), 2'-chloro-5'-fluoro-7',8'-fused phenyl- 1,4-dichloro-6-carboxyfluorescein (NED), 2'-chloro-7'-phenyl-1,4-dichloro-6-carboxyfluorescein (VIC), fluorescein isothiocyanate (FITC), 5,6-carboxymethyl fluorescein, Texas red, nitrobenz-2- oxa-1,3-diazol-4-yl (NBD), coumarin, dansyl chloride, amino-methyl coumarin (AMCA), Erythrosin, BODIPY dye, CASCADE BLUE dye, OREGON GREEN dye, pyrene, lissamine, xanthenes, acridines, oxazines, phycoerythrin, QUANTUM DYE, thiazole orange-ethidium heterodimer, and the like.

Suitable quenchers for use in the FRET cassettes of the disclosed technique include, but are not limited to: cyanine dyes, such as Cy3, Cy3.5, Cy5, Cy5.5, and Cy7, rhodamine dyes, such as tetramethyl-6-carboxyrhodamine (TAMRA) and tetrapropano-6- carboxyrhodamine (ROX), DABSYL dye, cyanine dyes, nitrothiazole blue (NTB), anthraquinone, malachite green, nitrothiazole, or nitroimidazole compounds, QSY7 (Molecular Probes, Eugene, OR), Eclipse quencher (Epoch Biosciences, Inc., Logan UT), and the like. Alternative quenchers include Black Hole Quencher dyes, particularly BHQ-1, BHQ-2, and BHQ-3 (Biosearch Technologies, Petaluma CA); BlackBerry® Quenchers (Berry & Associates, East Dexter, MI); and Iowa Black® (Integrated DNA Technologies, Coralville, IA). Analysis of factors such as absorbance and emission spectra of various molecules in selection of pairs or groups of moieties for use in FRET configurations is well known to those of skill in the art.

### Temperature-Dependence of the Invasive Cleavage Assay

FIG. 2 illustrates the temperature profile of a typical invasive cleavage assay carried out using oligonucleotide components diagramed in FIG. 1A, where the primary and secondary reactions are designed to take place simultaneously at the same reaction temperature. Generally speaking, the temperature optimum (*i.e.,* corresponding to the maximum of the curve shown in FIG. 2) for an invasive cleavage reaction will occur at or within 3°C the Tm of the cycling probe (*e.g.,* primary probe or cleaved 5' flap). The same guideline applies to an invasive cleavage assay where the primary and secondary invasive cleavage reactions take place under different optimal temperature conditions. In the instance illustrated in FIG. 2, the Tm for hybridization of the invasive probe to the target nucleic acid was approximately 68°C. The Tm for hybridization of the primary probe to the target nucleic acid was approximately 62°C. The Tm for hybridization of the cleaved 5' flap to the FRET cassette was approximately 62°C. When the signal generating reaction was carried out using standard reagents and procedures at different temperatures, and then fluorescent endpoint readings compared (*i.e.,* as signal-to-noise ratios) with results from similar reactions that did not include target nucleic acid, the bell-shaped curve shown in the figure was obtained. Again, the key finding was that maximal reaction activity substantially took place at the Tm, which corresponded to the peak of the curve. As well, shoulders on the curve were steep, causing declines to about background at about 5°C above and below the Tm, or peak of the curve. The assay clearly was temperature-dependent, and generated maximal fluorescent signal when the reaction temperature was within ± 3°C of the Tm of the oligonucleotide(s) that cycled in the reaction. In this example, the target annealing region of primary probe, and the cleaved 5' flap cycled on and off the target nucleic acid and FRET cassette, respectively. We drew from this that two invasive cleavage reactions, having curve peaks spaced apart by 5-10°C could be conducted independently at or about temperatures corresponding to the respective Tms in the same reaction mixture.

As illustrated in FIG. 2, the invasive probe remains hybridized to the target nucleic acid at or below the temperature optimum for the invasive cleavage assay. In this example, but not in all instances, the invasive probe does not remain bound to the target nucleic acid at temperatures substantially above the temperature optimum for the invasive cleavage assay. Cycling of oligonucleotides also is dependent on the assay temperature profile, such that, in this example, the cleaved 5' flap and the primary probe undergo cycling at the temperature optimum. Substantially below the temperature optimum the oligonucleotides do not cycle because they remain stably bound to their respective complementary nucleic acids. Substantially above the temperature optimum, the oligonucleotides do not cycle because they cannot stably anneal to their respective complements.

### Multiplex Detection

The present disclosure provides for the use of multiplex invasive cleavage assays, where a plurality of target nucleic acid sequences contained in a test sample can be detected in a single reaction mixture, using single channel detection of as few as a single type of fluorescent moiety as the reporter. In some examples, the multiplex invasive cleavage assays are quantitative assays that again involve detection of multiple target nucleic acid sequences in a single reaction mixture using detection of a single type of fluorescent moiety as the reporter.

In accordance with the disclosure, preparing a multiplex invasive cleavage assay reaction mixture involves combining (*e.g.,* into a single reaction vessel) oligonucleotide reagents of at least two separate invasive cleavage assays, where each assay operates with a different temperature optimum, and where signals generated in each reaction are detected using the same fluorescence detection channel, and preferably using the same fluorophore. A FEN enzyme further is included. In some examples, these multiplex assays combine into a single reaction mixture the reagents for at least two different invasive cleavage assays, where the temperature optima for conducting primary and secondary reactions for each of the assays differ from the temperature optima for conducting primary and secondary reactions of other assays in the multiplex by at least 5-30°C, more preferably by about 10-30°C, still more preferably by 10-20°C, or even by 10-15°C. In other examples, these multiplex assays combine into a single reaction mixture the reagents for at least two different invasive cleavage assays, where the temperature optima for conducting primary reactions for each of the assays are substantially similar, but where temperature optima for conducting secondary reactions of the different assays differ by at least 5-30°C, more preferably by about 10-30°C, still more preferably by 10-20°C, or even by 10-15°C. In some examples, three, four, or even five or more different invasive cleavage assays can be multiplexed.

Each invasive cleavage assay of the multiplex includes two target sequence-specific, unlabeled oligonucleotides for the primary reaction: an upstream invasive probe oligonucleotide, and a downstream primary probe oligonucleotide. The invasive probe oligonucleotide is generally designed for stable hybridization to the target nucleic acid at the temperature used for conducting the primary cleavage reaction, while the primary probe is designed to freely associate and disassociate with the target strand under the same temperature conditions. The primary probe includes a 5' flap sequence that is not complementary to the target nucleic acid contained in the test sample (*see* FIG. 1). The primary cleavage reaction occurs only when an uncut primary probe hybridizes adjacent to an overlapping invasive probe oligonucleotide. Each invasive cleavage assay further includes a FRET cassette having a first dye (*e.g.,* a fluorophore) and a second dye (*e.g.,* a quencher) in energy transfer relationship such that emission from the fluorophore is quenched when the two moieties are joined to the same FRET cassette. In some examples, the same fluorophore will be used in instances where multiple FRET cassettes are present for detecting different targets in the multiplex.

Detecting different target nucleic acids in the multiplex assay, and distinguishing each from the other, involves monitoring and/or quantifying detectable signal produced while the reaction mixture is incubated at the various temperature optima. For example, incubating a multiplex reaction at the temperature optimum for a first invasive cleavage assay (specific for a first target nucleic acid) can produce detectable signal if the first target nucleic acid is present in the reaction mixture. The quantity of signal produced will be proportional to the amount of the first target nucleic acid present. At least the secondary reaction of a multiplexed second invasive cleavage assay having a different temperature optimum (*i.e.*, being different from the temperature optimum of the first invasive cleavage assay by at least 5°C, more preferably by 5-30°C, more preferably 10-30°C, still more preferably 10-20°C, and still yet more preferably 10-15°C) will be substantially inactive at the time the first invasive cleavage assay is active. Accordingly, signal measured while the multiplex reaction is incubated at the first temperature optimum will only reflect activity of the first invasive cleavage assay. Following a shift in reaction temperature to the temperature optimum for the second invasive cleavage assay, the first invasive cleavage assay will be substantially inactive. Signal generated by the second cleavage assay will be cumulative to any signal generated in the multiplex reaction mixture when the first invasive cleavage assay was active. Accordingly, the quantity of signal measured at the conclusion of the first invasive cleavage assay (or alternatively after conclusion of the first invasive cleavage assay, but before the second invasive cleavage assay substantially has begun to generate signal) will be useful for indicating the presence of the first target nucleic acid. Likewise, the presence of the second target nucleic acid will be indicated by the quantity of signal measured at the conclusion of the second invasive cleavage assay, after subtracting the contribution of signal generated by the first invasive cleavage assay.

One aspect of the disclosed technique involves determining from measured signals whether or not a particular nucleic acid is present in the test sample. This is done by comparing the magnitude of the measured signal with a threshold value or threshold criterion. An exemplary threshold value isa static threshold value. For example, a threshold criterion of 5,000 RFU may be established for determining the presence or absence of a target nucleic acid. A measured signal exceeding this value can indicate the sample contains the target nucleic acid of interest. A measured signal below this value can indicate an insufficient amount of target was present to produce the necessary signal, and a determination can be made that the sample did not contain the target nucleic acid of interest. Of course, the presence or absence of different target nucleic acids in a multiplex invasive cleavage assay can be determined by comparison of the different signal magnitudes with different threshold criteria. Typically, signal magnitudes associated with an invasive cleavage assay *(e.g.,* a fluorescent signal generated in a secondary reaction) for a particular target will be measured at the end of a reaction period, preferably after the secondary reaction has stopped but before the signal has begun to increase from an invasive cleavage assay (*e.g.,* a fluorescent signal generated in a secondary reaction) for a different target nucleic acid being detected in the multiplex assay. It should be clear that measured fluorescence can increase in the reaction mixture as each secondary reaction for a different target nucleic acid takes place.

An alternative approach for determining the presence or absence of a target nucleic acid in a test sample involves comparing the time-dependent rate of fluorescence accumulation (sometimes referred to as "velocity") against a threshold criterion. In this instance, it may be required that the calculated velocity exceed a certain minimum threshold velocity for a test sample to be judged as containing a target nucleic acid of interest. A calculated velocity below the threshold velocity value could be judged as indicating absence of the target nucleic acid. Again, determination about the presence or absence of a target nucleic acid involves comparison with a threshold criterion.

Generally speaking, and in view of the foregoing discussion, a measured signal can be said to exceed a threshold in either of two ways. First, the measured signal can be assessed in terms of its magnitude, where the magnitude of the measured signal either exceeds (*i.e*., is greater than) or does not exceed *(i.e.,* is less than) a threshold magnitude value. As indicated above, exceeding the threshold preferably indicates the presence of the target nucleic acid, while falling below the threshold preferably indicates its absence. Second, a measured signal can be measured over a known or fixed period of time, in which case a velocity can be calculated. Here the calculated velocity can be compared against a threshold velocity required to establish the presence of the target nucleic acid in a test sample. More particularly, a velocity that exceeds (*i.e.,* is greater than) or does not exceed (*i.e.,* is less than) a velocity threshold value can be used for determining the presence or absence of a target nucleic acid. As indicated above, a velocity exceeding the threshold preferably indicates the presence of the target nucleic acid, while a velocity falling below the velocity threshold preferably indicates its absence. Both approaches are illustrated in the working Examples.

### Use of Equivalent Donor Moieties for Labeling FRET Cassettes

Generally speaking, the first and second FRET cassettes used in a multiplex assay for detecting different target sequences should include donor moieties (e.g., fluorophores) that are equivalent, and possibly identical. This facilitates single channel detection indicating that a secondary cleavage reaction has taken place. More particularly, the moieties to be detected by fluorescent emission in a single channel of a detection instrument will be equivalents *(i.e.,* equivalent fluorophores). Whether the equivalent donor moieties are excited directly by an external energy source, or whether they receive energy from a nearby second moiety in energy transfer relationship is a matter of design choice. Notably, details of energy transfer from a first fluorophore to a second fluorophore, followed by emission of a detectable wavelength from the second fluorophore, are discussed in U.S. 6,150,097.

Two donor moieties that are equivalent can be excited by a common (*i.e.,* shared) wavelength range, and may exhibit different maxima in their emission spectra, while emitting with measurable intensities over an arbitrary detection range. In this respect, the different donors (or moieties producing detectable fluorescent emissions) being detected advantageously exhibit spectral emission "crosstalk" (sometimes referred to as "bleed-through" or "crossover"). The ordinary skilled artisan will appreciate this feature of equivalent donors is ordinarily undesirable, and so is avoided, when different labeled probes are to be distinguished by their emission wavelengths. In some examples, emission spectra of equivalent donors will be overlapping over the range of emission wavelengths by at least 50%, more preferably by at least 60%, more preferably by at least 70%, more preferably by at least 80%, and still more preferably by at least 90%. Identical donors (*i.e*., the first and second FRET cassettes each being labeled with the same donor) exhibit 100% overlap. For example, the equivalent Cy3 and Alexa Fluor® 555 (registered trademark of Molecular Probes, Inc.) dyes are known to share similar excitation profiles, and virtually identical (*i.e.,* approaching 100%) emission profiles.

As indicated above, fluorescent emissions from equivalent donors can be detected in a single channel of a fluorescence monitoring instrument. This can be accomplished by selecting optical filter sets, or tunable wavelength ranges, of fluorescence monitoring instruments that permit transmission of wavelengths generated by the different donor moieties. In some examples, when different FRET cassettes are labeled with different donor moieties (e.g., different fluorophores) it is preferred that the different donors have emission wavelength maxima that differ by 0 nm to 60 nm, more preferably by 0 nm to 50 nm, more preferably 0 nm to 40 nm, more preferably 0 nm to 30 nm, more preferably 0 nm to 20 nm, or more preferably 0 nm to 10 nm. The ordinary skilled artisan will appreciate that various combinations of donor moieties will be considered equivalent in the context of the techniques disclosed herein. For example, a first FRET cassette can be labeled with a ROX donor dye (excitation maximum of 575 nm; emission maximum of 602 nm), and a second FRET cassette can be labeled with Alexa Fluor® 568 (registered trademark of Molecular Probes, Inc.) dye (excitation maximum of 578 nm; emission maximum of 603 nm). Thus, donor moieties that are equivalent, but not identical, are useful for labeling FRET cassettes that can be used in combination according to the disclosed technique.

The simplest approach to follow when designing FRET cassettes having equivalent donors and acceptors involves labeling the different FRET cassettes with identical donor moieties (*e.g.*, identical fluorophores) and identical acceptor moieties (*e.g.*, identical quenchers).

### Design of Oligonucleotides for Use in Multiplex Invasive Cleavage Assays

The multiplex technique disclosed herein takes advantage of flexibility in the design of oligonucleotide components of invasive cleavage assays. Invasive cleavage reactions are temperature-dependent, and generate increasing signal as a function of time when the temperature used for performing the reaction substantially corresponds to the Tm of the oligonucleotide sequence that is designed to cycle (*i.e.,* the sequence of the primary probe that hybridizes to target nucleic acid; and the sequence of the cleaved 5' flap that anneals to the FRET cassette). Cycling does not substantially occur at about 5°C below this Tm, and so there is substantially no signal generation. At about 5°C above this Tm the oligos will not substantially anneal, and so there is substantially no signal generation. By this approach the activities of multiplexed invasive cleavage reactions can be separated in time when the reaction temperature is shifted and then maintained constant for a period of time during which signal indicative of a particular target sequence is collected.

Stability of hybridization between the invasive probe and the target nucleic acid at the temperature used for carrying out the primary cleavage reaction is controlled using Tm as a criterion. More particularly, invasive probe oligonucleotides (which do not cycle on and off the target nucleic acid during the invasive cleavage assay) generally are designed to hybridize to the target nucleic acid with a Tm that is 5-20°C, more preferably 5-15°C higher than the Tm for hybridization of the primary probe to the target nucleic acid. This means the invasive probe will remain substantially hybridized when the reaction is conducted at a temperature corresponding to the Tm for binding of the primary probe to the target nucleic acid.

In certain examples, the primary probe for a particular invasive cleavage assay in the multiplex hybridizes to the target nucleic acid with a Tm that is substantially similar to the Tm for hybridization of the cleaved 5' flap (*i.e*., being cleaved from the primary probe) to the FRET cassette used for detecting that cleaved flap. The Tms are preferably within 2°C, more preferably within 1°C of the intended temperature optimum for the reaction(s). When this is the case, the primary cleavage reaction and the secondary cleavage reaction will share similar temperature profiles. Both the primary and secondary reactions for detection of a particular target nucleic acid will individually undergo linear cycling reactions, giving rise to cleaved 5' flap and cleaved FRET cassette under the same set of temperature conditions (*i.e*., substantially corresponding to the Tm of the primary and secondary invasive cleavage reactions). Likewise, both the primary and secondary cleavage reactions of a particular invasive cleavage assay will be substantially inactive when the reaction temperature is raised or lowered by at least 5°C or 10°C.

In certain examples, the primary probes for a plurality of invasive cleavage assays in the multiplex hybridize to their respective target nucleic acids with Tms that are substantially similar to each other. The Tms are preferably within 2°C, more preferably within 1°C of the intended temperature optimum for the reactions. This arrangement permits a plurality of primary invasive cleavage reactions under a single temperature condition to generate cleaved 5' flaps that specifically indicate the presence of the respective corresponding target nucleic acids. Very importantly, the Tms for hybridization of the different cleaved 5' flaps to the FRET cassette(s) used for detection of those cleaved 5' flaps must differ from each other by at least 5°C, more preferably by 5-30°C, still more preferably by 10-30°C, still more preferably by 10-20°C, and yet still more preferably by 10-15°C. When this is the case the secondary cleavage reactions will not both be active under the same reaction temperature condition in the same reaction mixture. When one of the secondary reactions is active, the other(s) will be inactive.

Design and selection of oligonucleotides in accordance with the disclosure can be aided by existing software programs. For example, U.S. 2006/0147955 A1 describes a software application (called, "INVADER CREATOR") that can be used to design the invasive probe (*i.e*., the "INVADER oligonucleotide") and downstream primary probe that will hybridize to a chosen target region. The software calculates an estimated Tm for the probes, which can be used for generating candidate invasive cleavage assays for multiplexing. Of course, the same procedures can be applied to the design of 5' flap sequences and matched FRET cassettes.

Once the invasive probe, the primary probe having a target non-complementary 5' flap, and the FRET cassette have been designed, the oligonucleotides can be synthesized and subjected to routine testing to verify the intended performance characteristics. More specifically, the invasive probe and primary probe can be combined with the corresponding target nucleic acid, a FRET cassette, a FEN enzyme, and other reagents to verify the Tm profile of the overall assay. Exemplary reaction conditions will be familiar to those having an ordinary level of skill in the art.

### Temperature-Dependent Multiplexing: Temporal Isolation of Multiplex Reactions

General features of assay design can be understood with reference to the example illustrated in FIG. 3. The figure illustrates three reaction temperature conditions, and schematizes the status of three multiplexed invasive cleavage assays (*e.g.*, oligonucleotide reagents for conducting the multiplex assays), where each of the three assays detects a different target nucleic acid. The FRET cassettes used for detecting signals indicating the presence of the different targets are all labeled with the same fluorophore and quencher pairs. The three different invasive cleavage assays (*i*.*e*., the "first," "second," and "third" invasive cleavage assays) are shown as having temperature optima at 63°C, 53°C and 43°C, respectively. In this illustration both the primary and secondary cleavage reactions associated with detection of a particular target nucleic acid take place at the same temperature. Indeed, the primary and secondary cleavage reactions in this illustration are not conducted under different temperature conditions.

The left panel of FIG. 3 shows that only the first invasive cleavage assay actively generates detectable fluorescent signal under the first temperature condition (*i.e.,* being illustrated as the 63°C reaction temperature). More specifically, the primary and secondary cleavage reactions of the first assay take place, and fluorophore is released by cleavage of the first FRET cassette. For discussion purposes, the Tm for hybridization of the first primary probe to its complementary target nucleic acid, and the Tm for hybridization of the first cleaved 5' flap to the first FRET cassette may each be about 63°C. As illustrated under the first temperature condition, neither the second nor third invasive cleavage assay is active because oligonucleotides required to promote cleavage reactions for those assays do not hybridize to their corresponding target nucleic acids or FRET cassettes. Fluorescent signal measured while the reaction is maintained under the first temperature condition is used to determine the presence of absence of the first target nucleic acid in the sample undergoing testing. Accumulated fluorescent signal at the conclusion of the first invasive cleavage assay in the multiplex is represented by the bar shown in the lower left corner of the left panel of the figure.

The center panel of FIG. 3 shows the effect of shifting the temperature to the second temperature condition (*i.e.,* being illustrated as the 53°C reaction temperature). Only the second invasive cleavage assay actively generates detectable fluorescent signal. Here the primary and secondary cleavage reactions of the second assay (but not the first or third assays) are taking place, and fluorophore released by cleavage of the second FRET cassette is detected as an increase above the fluorescent signal generated by the first invasive cleavage assay that took place under the first temperature condition. For discussion purposes, the Tm for hybridization of the second primary probe to its complementary target nucleic acid, and the Tm for hybridization of the second cleaved 5' flap to the second FRET cassette may each be about 53°C. As illustrated under the second temperature condition, the first invasive cleavage assay is substantially inactive, and does not generate any fluorescent signal. This is because the first primary probe and the first cleaved 5' flap remain stably hybridized to the first target nucleic acid and the first FRET cassette, respectively. These oligonucleotides cannot participate in cycling hybridization reactions needed for increasing the amount of cleavage reaction products. The third invasive cleavage assay also is inactive under the second temperature condition (53°C) because the reaction temperature is high enough that the third primary probe cannot anneal to its complementary sequence to participate in the cycling reaction needed to generate cleaved 5' flaps. Accumulated fluorescent signal at the conclusion of the second invasive cleavage assay (*i.e.,* representing signal accumulated during the first and second invasive cleavage assays of the multiplex) is represented by the bar shown in the lower left corner of the center panel of FIG. 3.

The right panel of FIG. 3 shows the effect of shifting the temperature to the third temperature condition (*i.e.,* being illustrated as the 43°C reaction temperature). Only the third invasive cleavage assay actively generates detectable fluorescent signal. Here the primary and secondary cleavage reactions of the third assay (but not of the first or second assays) are taking place, and fluorophore released from cleavage of the third FRET cassette is detected as an increase above the fluorescent signal generated by the first and second invasive cleavage assays that took place under the first and second temperature conditions, respectively. For discussion purposes, the Tm for hybridization of the third primary probe to its complementary target nucleic acid, and the Tm for hybridization of the third cleaved 5' flap to the third FRET cassette may each be about 43°C. As illustrated under the third temperature condition, the first invasive cleavage assay remains substantially inactive, and does not generate fluorescent signal. This is because the first primary probe and the first cleaved 5' flap remain stably hybridized to the first target nucleic acid and the first FRET cassette, respectively. The second invasive cleavage assay also is substantially inactive, and does not generate any fluorescent signal. This is because the second primary probe and the second cleaved 5' flap are stably hybridized to the second target nucleic acid and the second FRET cassette, respectively. Thus, oligonucleotides of the first and second invasive cleavage assays cannot participate in cycling hybridization reactions needed for increasing the amount of cleavage reaction products. Accumulated fluorescent signal at the conclusion of the third invasive cleavage assay is represented by the bar in the lower left corner of the right panel of FIG. 3.

It should be clear from the foregoing discussion that fluorescent signal associated with each different multiplexed assay can easily be determined, even though only a single fluorophore species is used to generate the signal. In some examples, signal quantity resulting from the secondary reaction of the first invasive cleavage assay (before any subsequent secondary reaction is substantially initiated by a temperature shift) can be used to determine the presence or absence of the first target nucleic acid in a test sample. Signal quantity resulting from the secondary reaction of the second invasive cleavage assay can be determined by measurement at the conclusion of the second invasive cleavage assay followed by subtraction of the signal quantity due to the first invasive cleavage assay; or alternatively by monitoring signal accumulation as a function of time to determine the increase above the signal due to the first invasive cleavage assay. The signal quantity specifically due to the second invasive cleavage assay can be used to determine the presence or absence of the second target nucleic acid in the test sample. Signal due to the third invasive cleavage assay can be determined by measurement at the conclusion of the third invasive cleavage assay followed by subtraction of the signal quantity due to the first and second invasive cleavage assays; or alternatively can be measured as a function of time to determine the increase above signal due to the first and second invasive cleavage assays. The signal quantity specifically due to the third invasive cleavage assay can be used to determine the presence or absence of the third target nucleic acid in the test sample.

Finally, it should also be clear that, regardless of the number of different FRET cassettes used for detecting fluorescent signal indicating the presence of a particular target nucleic acid, a single fluorescent moiety (e.g., fluorescein) will be sufficient to permit detection of the different target nucleic acids in a manner that permits unique detection of each of the targets.

### Multiplex Invasive Cleavage Assays Employing a Common Primary Reaction Temperature Optimum, and Distinct Secondary Reaction Temperature Optima

In some examples, reagents for two or more different invasive cleavage assays are combined in a single reaction mixture, their respective primary reactions are carried out simultaneously (*i.e*., at the same time and under the same temperature conditions), and their respective secondary reactions are carried out at different times under different temperature conditions. Cleaved 5' flaps arising from the different primary reactions hybridize with different Tms to their respective FRET cassette(s), each harboring the same fluorophore moiety. Performing the secondary cleavage reactions at their respective temperature optima permits the two or more invasive cleavage assays to be monitored independently, as illustrated in FIG. 4. At least two different results can be obtained using the single fluorophore as a reporter, and a single detection channel of a fluorescence-monitoring instrument (e.g., a fluorometer). Still further, even though a plurality of target-specific primary reactions may occur at the same time, and at the same temperature, we can still obtain quantitative results for all targets using determined velocities. Velocity is governed by the enzyme kinetics, and the initial rate of the enzyme is dependent on substrate concentration. Therefore, the rate at which FRETs are cleaved is dependent on the number of flaps created in the primary reaction, which in turn is dependent on the concentration of target. Even when multiple primary reactions are combined, quantitative differences are still reflected in the secondary reactions.

FIG. 4 illustrates oligonucleotide reagents for conducting two different invasive cleavage assays in a multiplex format. The upper left portion of the figure shows a first target nucleic acid (*e.g.,* of human origin) hybridized to a first invasive probe and a first primary probe, where the Tm for hybridization of the first primary probe to the first target nucleic acid is 64°C. Preferably, the first invasive probe hybridizes to the first target with a Tm that is 5-20°C, more preferably 5-15°C higher than this value to ensure stable hybridization under the temperature conditions used for carrying out the primary reaction. The upper right portion of the figure shows hybridization of the first cleaved 5' flap (*i.e*., generated by a cleavage event in the first primary reaction) to a first FRET cassette, where the hybridization is characterized by a Tm of 64°C. The lower left portion of the figure shows a second target nucleic acid (*e.g.,* of bacterial origin; Prevotella) hybridized to a second invasive probe and a second primary probe, where the Tm for hybridization of the second primary probe to the second target nucleic acid is also 64°C. Preferably, the second invasive probe hybridizes to the second target with a Tm that is 5-20°C, more preferably 5-15°C higher than this value to ensure stable hybridization under the temperature conditions used for carrying out the primary reaction. The lower right portion of the figure shows hybridization of the second cleaved 5' flap *(i.e.,* generated by a cleavage event in the second primary reaction) to a second FRET cassette, characterized by a Tm of 43°C. In some examples, the structures of the first and second FRET cassettes are identical. What is important however, is that the structures of the first and second cleaved 5' flaps are different. This ensures that the first and second cleaved 5' flaps hybridize to the FRET cassette(s) with different Tms. In this example the Tms of the two cleaved 5' flaps differ by 21°C, which is consistent with preferred differences of at least 5°C, more preferably 5-30°C, and more preferably 10-30°C.

An exemplary reaction mixture including human and *Prevotella* bacterial target nucleic acids, first and second invasive probes, first and second primary probes, first and second FRET cassettes (each harboring the same interactive label pair), and FEN enzyme is useful for independently detecting the two target nucleic acids. Incubating the reaction mixture for a period of time at 64°C promotes the primary cleavage reactions for both of the three-member constructs illustrated in FIG. 4, thereby producing cleaved 5' flaps from both of the human- and bacterial-specific primary probes. As well, fluorophore cleaved from the first FRET cassette during the 64°C incubation optionally can be detected and quantified as an indication of the presence of the first target nucleic acid (e.g., human nucleic acid) in the reaction mixture. However, fluorophore is not cleaved from the second FRET cassette during the 64°C incubation because the second cleaved 5' flap has a Tm that is at least 5°C lower, and so annealing and cycling does not substantially occur. Shifting the reaction mixture to 43°C for a period of time suppresses further cleavage of the first FRET cassette, but promotes a cycling interaction between the second cleaved 5' flap and the second FRET cassette. As a result, fluorophore cleaved from the second FRET cassette can be detected and quantified as an indication of the presence of the second target nucleic acid in the reaction mixture.

Fluorescent signal produced during the different temperature steps can be used for independently detecting each of the different target nucleic acids. Fluorescent signal produced at the end of the 64°C temperature step, or alternatively after shifting the temperature by at least 5°C, more preferably by at least 10°C, but in any case before the secondary reaction of the second cleaved 5' flap and the second FRET cassette has substantially begun, can be used for establishing the presence of the first target nucleic acid in the reaction mixture. Fluorescent signal produced during the 43°C incubation step can be used for establishing the presence of the second target nucleic acid in the reaction mixture.

### Assays Integrating Real-Time Nucleic Acid Amplification with Detection Using Multiplex Invasive Cleavage Assays

Multiplexed invasive cleavage assays, employing only a single fluorophore and/or detection channel of an automated instrument, advantageously can be used to carry out real-time monitoring of a plurality of nucleic acid amplification reactions (*e.g.*, PCR reactions). By this approach two, three, four, and even five real-time run curves can be obtained for each fluorescent dye employed. A conventional four-channel real-time PCR instrument, when configured by appropriate software for executing the methods disclosed herein, can generate 8-20 real-time run curve results from a single reaction mixture.

In some examples, at least one amplified target nucleic acid being monitored as a function of reaction cycle or time in a reaction mixture originates from an analyte target nucleic acid contained in a sample undergoing testing. Preferably, the reaction mixture further includes amplified or non-amplified target nucleic acids, detected in the multiplex invasive cleavage assay, that serve as controls or calibration standards of known concentration of copy level. For example, one target nucleic acid being amplified can be a calibration standard added to the reaction mixture at a known copy level. Alternatively, two target nucleic acids being amplified can be calibration standards added to the reaction mixture at known copy levels. The calibration standards will be distinguishable from each other, and from the analyte target nucleic acid, for example by the presence of single nucleotide differences.

There are several options for carrying out integrated assays and analyzing outcomes. Broadly speaking, there are two general formats that can be followed: (1) post-PCR analysis of amplification products using multiplex invasive cleavage reactions for amplicon detection (*i.e,* so called "endpoint" detection); and (2) concurrent monitoring of a plurality of nucleic acid reactions using multiplex invasive cleavage assays as the amplification reactions are taking place (*i.e*., so called "real-time" monitoring). These two formats can be used individually, or used in combination in the same assay.

An example of endpoint detection would be a PCR reaction generating one or a plurality of amplification products in a first stage, followed by detection of the amplification product(s) after the amplification reaction was complete. This could include detection of a plurality of different targets, or a plurality of different sequences within the same amplified target nucleic acid. The multiplex invasive cleavage assay would involve detection of a plurality of different sequences, each being detected independently using only a single fluorophore and/or detection channel.

An example of real-time monitoring would be a multiplex PCR reaction generating a plurality of different amplification products, with monitoring of a plurality of amplified target nucleic acids while the reaction was taking place (*e.g.*, during discrete cycles of the amplification reaction), using multiplex invasive cleavage assays. Again, each amplification product being monitored would be detected independently using only a single fluorophore and/or detection channel.

An example of a combination format would be a multiplex PCR reaction generating a plurality of amplification products, with monitoring of at least one amplified target nucleic acid as the reaction was taking place *(i.e.,* real-time monitoring), together with detection of at least one other amplified target nucleic acid at the conclusion of the amplification reaction *(i.e.,* endpoint detection). Both the real-time monitoring and endpoint detection reactions can be carried out as multiplex invasive cleavage reactions in the same reaction mixture using only a single fluorophore and/or detection channel of an analytical instrument.

Integrated techniques involving time-dependent monitoring of nucleic acid amplification using multiplex invasive cleavage assays involve combining a plurality of invasive cleavage reactions that operate at different reaction temperatures, and then performing and assessing reactions according to a stepwise temperature profile with monitoring of a single fluorescent dye. FIG. 5 illustrates a model system wherein each of four target nucleic acid sequences (*i.e*., Factor II, MTHFR 677, Factor V; and MTHFR 1298) can be present in wild-type ("WT") or mutant ("Mut") forms (*i.e*., representing eight different target sequences). The left portion of FIG. 5 schematically illustrates reagents for different invasive cleavage assays, a different assay being specific for each of the different target nucleic acids. As indicated in the diagrams, sets of invasive cleavage assay reagents specific for wild-type target nucleic acids can harbor the same fluorophore-quencher pairs. Likewise, the set of invasive cleavage assay reagents specific for mutant genotypes can harbor fluorophore-quencher pairs that are the same as each other, but different from the pairs used for detection of wild-type sequences. The upward/downward arrows represent cycling hybridization/dissociation of the primary probe with its respective target nucleic acid; and cycling hybridization/dissociation of the cleaved 5' flaps with their respective FRET cassettes that include the interactive label pairs. Cycling occurs at the invasive cleavage reaction temperature optima, indicated in this model system to be: 72°C for the Factor II invasive cleavage assay; 63°C for the MTHFR 677 invasive cleavage assay; 53°C for the Factor V invasive cleavage assay; and 43°C for the MTHFR 1298 invasive cleavage assay.

Complete reaction mixtures (including target nucleic acid templates, PCR primers, DNA precursors *(i.e.,* dNTPs), oligonucleotide reagents for conducting the eight different invasive cleavage assays, a thermostable DNA polymerase, and a thermostable FEN enzyme) can be subjected to thermal cycling, with fluorescence monitoring at regular cycle intervals during the nucleic acid amplification procedure, followed by endpoint detection. Oligonucleotide reagents for conducting the invasive cleavage assays include: one invasive probe for each of four target nucleic acids (without needing to distinguish WT from Mut genotypes), one WT and one Mut primary probe for each of four target nucleic acids, and one FRET cassette for each of the eight different primary probes *(i.e.,* one WT and Mut for each of four target nucleic acids). Temperature cycling generally involves an initial high temperature incubation to denature double-stranded nucleic acids, followed by a series of cycles to permit primer annealing, primer extension, and at least one primary and secondary reaction to permit fluorescence signal development. Detection of target nucleic acids using multiplexed invasive cleavage reactions at the conclusion of the DNA polymerizing steps can take place either by performing primary and secondary reactions at a single temperature for each different target nucleic acid to be detected, or by permitting all primary reactions to occur at a single temperature, followed by stepped temperature cycles to permit individual secondary reactions to occur, or by permitting two or more primary reactions to occur at one or more temperatures, followed by stepped temperature cycles to permit individual secondary reactions to occur.

Processing of fluorescent signal data collected during the real-time and optional endpoint readings permits different target nucleic acids to be detected independently. In general terms, each different secondary reaction that takes place in the reaction mixture (*i.e*., in a sequential fashion using the same fluorophore), whether monitored in real-time or as an endpoint reading, adds to the level of fluorescence in a cumulative fashion. Measured fluorescence can be assigned to a particular secondary reaction and followed throughout the procedure. By subtracting contributions due to other (*e.g.*, preceding) secondary reactions in the multiplex invasive cleavage assay, it becomes possible to isolate fluorescence signals due to an individual reaction. This is true for assembling real-time and endpoint fluorescence data, both of which are demonstrated below in Example 2.

FIG. 6 illustrates how the four target nucleic acids can be amplified; shows the locations of PCR primers (shown as arrows); schematizes hybridization of invasive probes to the different target nucleic acids; and illustrates two primary probes and two FRET cassettes for use in each of the four invasive cleavage assays. One primary probe and the respective FRET cassette having a region complementary to the cleaved 5' flap of that primary probe are used for each of the wild-type and mutant sequences. The stepped temperature profiles illustrate which invasive cleavage assay will be active at a particular temperature. The four boxed real-time run curve schematic diagrams at the right side of the figure show results consistent with detection of wild-type Factor II, MTHFR 677, and MTHFR 1298; and with detection of the mutant version of Factor V. The Factor II nucleic acid target is detected by real-time monitoring in the same assay that detects MTHFR 677, Factor V, and MTHFR 1298 using end-point protocols. It will be apparent from examination of the different run curves that fluorescence appearing in a single channel is cumulative to the level of fluorescence detected in the prior step.

### Cycling of Temperatures

In some examples, sequential fluorescent readings taken during different temperature steps, corresponding to fluorescent signals generated by invasive cleavage reactions specific for different target nucleic acids, are taken as temperature shifts take place in order from higher temperatures to lower temperatures.

In some examples, a plurality of primary reactions of different invasive cleavage assays in a multiplex invasive cleavage assay take place at a common temperature *(i.e.,* the primary reactions share a common temperature profile) and different secondary reactions generate fluorescent signals indicative of the presence of different target nucleic acids, at different temperatures. When this is the case, the sequential steps of fluorescent readings preferably take place in order from higher temperature to lower temperature.

In some examples, when synthesis of a plurality of nucleic acid amplifications products is monitored at repeated cycles (*e.g.*, once every complete cycle of denaturation/primer annealing/primer extension/fluorescence reading; or even once every other cycle) during a nucleic acid amplification reaction, such as a PCR reaction, the primary reactions for multiplexed invasive cleavage assays (*e.g.*, two assays detecting different target nucleic acids in the same reaction mixture) can take place at a common temperature, such as the temperature used for primer extension, and the secondary reactions that generate fluorescent signals can take place at temperatures stepped lower than the temperature used for performing the primer extension reactions, where the different temperature steps are spaced apart by at least 5°C, more preferably by about 5-15°C, and still more preferably by 5-10°C. Differences between the temperature steps permits one secondary reaction in the multiplex to be carried out and monitored at a single time. Indeed, two secondary reactions indicative of the presence of different target nucleic acids should not occur at any single time, or during any single temperature step. By this approach two, three, and possibly even four or more invasive cleavage assays specific for different target nucleic acids, where each of the multiplexed assays uses the same fluorophore as an indicator of reaction progress, can be combined in a single reaction mixture, and monitored independently. With particular reference to PCR reactions, rapid increases from the lowest temperature used for reading a fluorescent signal to the temperature used for denaturation of double-stranded nucleic acids may permit small increases in the accumulated fluorescent signal, but are not known to affect validity of results. This is because the magnitudes of fluorescent signals generated during particular temperature steps in the cycle of reactions will be compared to respective baseline or background values at each step. In this way the amount of fluorescent signal associated with a particular step of a particular secondary reaction can be determined.

### Modifications Affecting Oligonucleotide Hybridization Characteristics

The ability to multiplex could be further enhanced by adjusting reaction conditions (oligo concentrations, enzyme level, buffer, reaction profile, *etc.).* This optimization may result in the ability to obtain at least 3, more preferably 4, and possibly 5 answers per fluorescent dye.

The present disclosure further provides methods for changing the hybridization and Tm profile characteristics of oligonucleotides (*i.e*., invasive probes, primary probes, and cleaved 5' flaps) used in invasive cleavage assays. By this approach it is possible to modulate temperature optima of multiplexed invasive cleavage assays, and so to maintain at least secondary reactions independent of each other. Certain exemplary techniques for altering oligonucleotides are provided below.

### i. Mismatch Oligonucleotides

In some examples, the present disclosure provides oligonucleotides with one or more (preferably one) mismatch with the corresponding target sequence at a position removed from any cleavage site by at least 4-6, or 10 or more nucleotides. The disclosed technique is not limited to a particular mechanism. Indeed, an understanding of the mechanism is not necessary to practice the disclosed technique. Nonetheless, it is contemplated that the presence of one or more mismatches allows the oligonucleotide to bind to the target, but with a reduced affinity as compared to a corresponding oligonucleotide lacking mismatches, thereby affecting the Tm for hybridization of an oligonucleotide to its target nucleic acid.

### ii. Charge Modified Oligonucleotides

In other examples, the disclosed technique utilizes charge modified oligonucleotides to alter binding efficiency (*See e.g.,* U.S. Pat. No. 6,780,982. In some examples, the charge modified oligonucleotides comprise "charge tags." Positively charged moieties need not always carry a positive charge. As used herein, the term "positively charged moiety" refers to a chemical structure that possesses a net positive charge under the reaction conditions of its intended use (*e.g.*, when attached to a molecule of interest under the pH of the desired reaction conditions). Indeed, in some examplesof the present disclosure, the positively charged moiety does not carry a positive charge until it is introduced to the appropriate reaction conditions. This can also be thought of as "pH-dependent" and "pH-independent" positive charges. pH-dependent charges are those that possess the charge only under certain pH conditions, while pH-independent charges are those that possess a charge regardless of the pH conditions.

The positively charged moieties, or "charge tags," when attached to another entity, can be represented by the formula: X--Y where X is the entity *(e.g.,* a nucleic acid molecule, *etc.)* and Y is the charge tag. The charge tags can be attached to other entities through any suitable means (*e.g.*, covalent bonds, ionic interactions, *etc.)* either directly or through an intermediate (*e.g.,* through a linking group). In an example, where X is a nucleic acid molecule, the charge tag is attached to either the 3' or 5' end of the nucleic acid molecule.

The charge tags may contain a variety of components. For example, the charge tag Y can be represented by the formula: Y₁--Y₂ where Y₁ comprises a chemical component that provides the positive charge to the charge tag and where Y₂ is another desired component. Y₂ may be, for example, a dye, another chemical component that provides a positive charge to the charge tag, a functional group for attachment of other molecules to the charge tag, a nucleotide, *etc.* Where such a structure is attached to another entity, X, either Y₁ or Y₂ may be attached to X. X--Y₁--Y₂ or X--Y₂--Y₁.

The disclosed technique is not limited by the nature of the chemical component(s) that provide(s) the positive charge to the charge tag. Such chemical components include, but are not limited to, amines (primary, secondary, and tertiary amines), ammoniums, and phosphoniums. The chemical components may also comprise chemical complexes that entrap or are otherwise associated with one or more positively charged metal ions.

In an example of the present disclosure, charge tags are attached to nucleic acid molecules *(e.g.,* DNA molecules). The charge tags may be synthesized directly onto a nucleic molecule or may be synthesized, for example, on a solid support or in liquid phase and then attached to a nucleic acid molecule or any other desired molecule. In another example, charge tags that are attached to nucleic acid molecules comprise one or more components synthesized by H-phosphonate chemistry, by incorporation of novel phosphoramidites, or a combination of both. For example, compositions of the present disclosure include structures such as: [X]-[Y₁--Y₂--Y₃--Y₄] where [X] is a nucleic acid molecule and [Y...] is a charge tag. In one example, Y₁ is a dye, Y₂ is synthesized using H-phosphonate chemistry and comprises a chemical component that provides a positive charge to the charge tag, Y₃ is a positively charged phosphoramidite, and Y₄ is a nucleotide or polynucleotide. Any of the Y components are interchangeable with one another.

As discussed above, one or more components of a charge tag can be synthesized using H-phosphonate chemistry. Production of charge tag using the methods described herein provides a convenient and flexible modular approach for the design of a wide variety of charge tags. Since its introduction, solid phase H-phosphonate chemistry (B. C. Froehler, Methods in Molecular Biology, 20:33, S. Agrawal, Ed. Humana Press; Totowa, N.J. [1993]) has been recognized as an efficient tool in the chemical synthesis of natural, modified and labeled oligonucleotides and DNA probes. Those skilled in the art know that this approach allows for the synthesis of the oligonucleotide fragments with a fully modified phosphodiester backbone (*e.g.*, oligonucleotide phosphorothioates; Froechler [1993], supra) or the synthesis of oligonucleotide fragments in which only specific positions of the phosphodiester backbone are modified (Agrawal, et al., Proc. Natl. Acad. Sci USA, 85:7079 [1988], Froehler, Tetrahedron Lett. 27:5575 [1986], Froehler, et al., Nucl. Acids Res. 16:4831 [1988]). The use of H-phosphonate chemistry allows for the introduction of different types of modifications into the oligonucleotide molecule (Agrawal, et al., Froehler[1986], supra, Letsinger, et al., J. Am. Chem. Soc., 110:4470 [1988], Agrawal and Zamecnik, Nucl. Acid Res. 18:5419 [1990], Handong, et al., Bioconjugate Chem. 8:49 [1997], Vinogradov, et al., Bioconjugate Chem. 7:3 [1995], Schultz, et al., Tetrahedron Lett. 36:8407 [1995]), however the replacement of the phosphodiester linkage by the phosphoramidate linkage is one of the most frequent changes due to its effectiveness and synthetic flexibility. Froehler and Letsinger were among first to use this approach in the synthesis of modified oligonucleotides in which phosphodiester linkages were fully or partially replaced by the phosphoramidate linkages bearing positively charged groups (*e.g.*, tertiary amino groups; Froehler [1986], Froehler, et al., [1988], and Letsinger, et al., supra).

In some examplesof the disclosed technique, charge tags are generated using H-phosphonate chemistry. The charge tags may be assembled on the end of a nucleic acid molecule or may be synthesized separately and attached to a nucleic acid molecule. Any suitable phosphorylating agent may be used in the synthesis of the charge tag. For example, the component to be added may contain the structure: A-B--P where A is a protecting group, B is any desired functional group *(e.g.,* a functional group that provides a positive charge to the charge tag), and P is a chemical group containing phosphorous. In one example, B comprises a chemical group that is capable of providing a positive charge to the charge tag. However, in some examples, B is a functional group that allows post-synthetic attachment of a positively charged group to the charge tag.

In other examples, positively charged phosphoramidites (PCP) and neutral phosphoramidites (NP) are utilized to introduce both positive charge and structure modulation into the synthesized charge-balanced CRE oligonucleotide (*See e.g.,* U.S. Pat. No. 6,780,982).

Standard coupling protocol with the use of the phosphoramidite reagents (which are compatible with the chemical synthesis of oligonucleotides) is associated with the introduction, into the growing molecule, of one negative charge per each performed coupling step, due to the formation of the phosphodiester linkage.

### iii. Nucleic Acid Modification Agents

Any internal (*e.g*., to the oligonucleotide) or external agent that alters the hybridization strength of oligonucleotide binding is suitable for use with the methods and compositions of the present disclosure. This is the case provided it is not inhibitory, or has limited inhibitory effect, on invasive cleavage and/or PCR chemistries.

In some examples, the present disclosure provides oligonucleotides comprising intercalating agents. Intercalating agents are agents that are capable of inserting themselves between the successive bases in DNA. In some examples, intercalating agents alter the binding properties of nucleic acids.

Examples of intercalating agents are known in the art and include, but are not limited to, ethidium bromide, psoralen and derivatives, acridines, proflavine, acridine orange, acriflavine, fluorcoumanin, ellipticine, daunomycin, chloroquine, distamycin D, chromomycin, homidium, mithramycin, ruthenium polypyridyls, and anthramycin.

In other examples, minor groove DNA binding agents are utilized to modify (*e.g.*, increase or decrease) the hybridization efficiency of oligonucleotides. Examples of minor groove binding agents include, but are not limited to, duocarmycins *(See e.g.,* Boger, Pure & Appl. Chem., Vol. 66, No. 4, pp. 837-844), netropsin, bisbenzimidazole, aromatic diamidines, lexitropsins, distamycin, and organic dications, based on unfused-aromatic systems *(See e.g.,* U.S. Pat. No. 6,613,787).

In still further examples, modified bases are utilized to alter the hybridization efficiency of oligonucleotides. For example, in some examples, modified bases that include charged groups are utilized. Examples include, but are not limited to, the substitution of a "T" nucleotide with "amino-T" in modified oligonucleotides.

In yet other examples, one or more nucleotides of an oligonucleotide are modified by the covalent attachment of groups that alter the hybridization properties of the oligonucleotide. Examples include, but are not limited to, the attachment of amino acids to nucleotides.

In yet other examples, oligonucleotides with base analogues are utilized to alter the hybridization characteristics. For example, nucleotides that do not form hydrogen bonds but that still participate in base stacking are utilized. Examples include but are not limited to non-polar, aromatic nucleoside analogs such as 2,4-difluorotoluene and "universal" bases such as 5-nitroindole and 3-nitropyrrole. In other examples, base analogs that retain hydrogen bonding ability are utilized (*See e.g*., US Patent application US20040106108A1 and WO 04/065550A3).

### iv. Oligonucleotide Length

In yet other examples, oligonucleotide length is altered in order to alter the hybridization characteristics of an oligonucleotide.

### v. Secondary Structure

In some examples, secondary structure is utilized to alter the hybridization efficiency of the oligonucleotide. For example, in some examples, two or more oligonucleotides are designed to hybridize the same target sequence. One of the oligonucleotides is designed to have minimal secondary structure. Additional oligonucleotides are designed that retain target sequence recognition, but that have secondary structure. It is contemplated that the oligonucleotides with secondary structure will exhibit altered hybridization properties.

### vi. Reaction Conditions

In still further examples, reaction conditions are modified to alter oligonucleotide hybridization characteristics. Examples of parameters that affect nucleic acid hybridization conditions include, but are not limited to, ionic strength, buffer composition, pH, and additives (e.g., glycerol, polyethylene glycol, and proteins).

### vii. Stacking Oligonucleotides

In still further examples, adjacently hybridizing oligonucleotides are used to alter oligonucleotide hybridization characteristics. When short strands of nucleic acid align contiguously along a longer strand, the hybridization of each is stabilized by the hybridization of the neighboring fragments because the base pairs can stack along the helix as though the backbone was, in fact, uninterrupted. This cooperativity of binding can give each segment a stability of interaction in excess of what would be expected for the segment hybridizing to the longer nucleic acid alone. In the event of a perturbation in the cooperative binding (*e.g.*, by a mismatch at or near the junction between the contiguous duplexes), this cooperativity can be reduced or eliminated. In some examplesof the present disclosure, oligonucleotides are configured to cooperate in distinct ways with one or more adjacently hybridizing oligonucleotides, so as to provide different hybridization characteristics. In some examples, an oligonucleotide comprises one or more mismatched bases at near the junction with the adjacent oligonucleotide, so as to alter or disrupt cooperativity of binding, as compared to an oligonucleotide lacking the mismatches. In other examples, an oligonucleotide comprises one or more base analogs selected to reduce stacking interactions with adjacent bases. In yet other examples, it is envisioned that gaps of one or more nucleotides (*e.g.*, by the use of truncated probes) are used to alter cooperativity and thus alter hybridization characteristics.

### viii. Other Approaches

Any other method for altering the hybridization of characteristics of an oligonucleotide may also be used with the disclosed technique. Other examples include, but are not limited to: use of sequences in oligonucleotides or targets that render the sequence susceptible to differential hybridization behavior in response to buffer conditions (*e.g.*, the use of guanosine-quartets) or protein/nucleic acid interactions (*e.g.*, by creating binding sites for nucleic acid binding proteins or enzyme that bind or alter nucleic acid sequences); use of dangling ends *(e.g.,* for dangling-end stabilization and stacking); attachment of iron or other magnetic agents to allow concentration of the nucleic acid in a magnetic field; use of agents that titrate out a specific oligonucleotide; and the like.

### Automated Systems for Conducting Multiplex Invasive Cleavage Assays

In some examples, the disclosed multiplex invasive cleavage assays are carried out using an automated nucleic acid analyzer that includes a temperature-controlled incubator and an attached fluorometer for monitoring samples contained therein. The fluorometer measures fluorescent emission, preferably as a function of reaction time.

Generally speaking, useful automated analyzers will include one or more temperature-controlled incubators (*e.g.*, either chambers or thermal blocks); an optical system that permits monitoring of reaction progress in a reaction mixture by detection of fluorescent emissions; a controller that directs changes in the position of reaction vessels and/or the temperature of thermal blocks; and a computer or processor that collects and analyzes fluorescent emission data and provides an output regarding the presence or amount of a plurality of target nucleic acids in the sample. In an example, the monitoring of reaction progress involves monitoring reaction progress as a function of time.

In another example, the automated system includes a plurality of temperature-controlled chambers, where each chamber maintains a substantially constant temperature, and where reaction vessels or receptacles are moved in and out of the chambers to alter the temperature of a reaction mixture contained therein. In another example, the automated system additionally performs the step of isolating and purifying a nucleic acid prior to performing the multiplex invasive cleavage assay using the isolated and purified nucleic acid for testing (*see e.g.,* U.S. 8,349,564). The Panther™ system (Hologic, Inc.) for automated *in vitro* diagnostic testing is one example of an instrument that can be used for carrying out techniques disclosed herein.

In another example, the automated system includes a single temperature-controlled chamber or thermal block that receives reaction vessels or receptacles, and that changes temperature to alter the temperature of a reaction mixture contained therein. The changes in temperature preferably are controlled by a programmable computer, which may be either an integral component of the thermal cycling device or a stand-alone programmable computer in communication with (*e.g*., via a wired or wireless connection) the thermal cycling device. Those having an ordinary level of skill in the art will be aware of numerous thermal cycling instruments and systems for conducting nucleic acid amplification reactions (*e.g*., PCR reactions), and will appreciate how those instruments and systems can easily be used for performing multiplex invasive cleavage assays by the disclosed technique. Examples of devices that may be used for carrying out the methods described herein include the Mx3005P™ real-time PCR system (Stratagene); and the LightCycler® 480 Instrument (Roche Diagnostics), both being plate-based real-time PCR devices useful for amplifying and detecting nucleic acids. Other systems that can be used include the Chromo4™ Real-Time PCR System (BioRad), which offers four color detection, and optional use of multiwell plates or tubes as reaction vessels. Still other systems include the Applied Biosystems 7500 Fast Real-Time PCR System (Applied Biosystems); and the ViiA™ 7 Real-Time PCR System (Life Technologies). These devices and systems include a thermal block for adjusting the temperature of reaction mixtures that remain in a fixed position while monitored reactions are performed.

Preferably, reaction mixtures for performing the multiplex invasive cleavage assays are complete by the time a first enzymatic reaction (*e.g.*, reverse transcription, 5' flap cleavage, FRET cassette cleavage, *etc*.) is initiated, and there is no need to add additional reagents prior to the step of detecting signals indicating the presence of each of the targets independent of the other. Thus, the method can be carried out in a reaction vessel (*e.g*., including closed and sealed reaction vessels) without the need for additional reagents after the first enzymatic reaction is initiated. In some examples, the method is carried out using genomic DNA that has not been amplified. In some examples, the method involves an invasive cleavage reaction that follows reverse transcription to create the DNA target for hybridization of the invasive probe and the primary probe. In some examples, the method involves an invasive cleavage reaction that follows a PCR reaction to create the DNA target for hybridization of the invasive probe and the primary probe. In some examples, because of the signal amplification, sensitivity, and ability to quantify signal using an invasive cleavage reaction, where the polymerase chain reaction is used only limited cycles need only to be used (*e.g*., at least one, but no more than 20, 15, 12, 10, or fewer cycles).

### Preferred Computer Control and Data Processing Systems

The methods disclosed herein are conveniently implemented using a computer or similar processing device ("computer" hereafter). In different examples, software or machine-executable instructions for conducting and analyzing multiplex invasive cleavage assays can be loaded or otherwise held in a memory component of a freestanding computer, or in a memory component of a computer linked to a device used for monitoring, preferably as a function of time, the magnitude of a fluorescent signal undergoing analysis. In another example, software for executing the multiplex invasive cleavage procedure is held in a memory component of a computer that is linked to, or that is an integral part of a device capable of monitoring a fluorescent signal generated in a reaction mixture as a function of time. Computer controlled instruments for performing and monitoring real-time nucleic acid amplification can be adapted to run multiplex invasive cleavage assays by modification with appropriate software instructions.

Either or both of a controller system for controlling a nucleic acid amplification device and/or the detection system of the real-time amplification device can be coupled to an appropriately programmed computer which functions to instruct operation of these instruments in accordance with preprogrammed or user input instructions. The computer preferably receives data and information from these instruments, and interprets, manipulates and reports this information to the user.

In general, the computer typically includes appropriate software for receiving user instructions, either in the form of user input into a set of parameter fields, or in the form of preprogrammed instructions (e.g., preprogrammed for a variety of different specific operations). Preprogrammed instructions may take the form of machine-readable codes, such as a bar code. The software converts these instructions to appropriate language for instructing operation of the real-time amplification controller to carry out the desired operation. The computer also is capable of receiving data from the one or more sensors/detectors included within the system, and interprets the data in accordance with the programming. The system preferably includes software that analyzes the magnitude and/or rate of change of fluorescence accumulated during performance of the different secondary reactions of invasive cleavage assays in the multiplex. In some examples, the software independently analyzes the magnitude of fluorescence accumulated, or alternatively the rate of fluorescence signal accumulation, during a period of time under a temperature condition where a single secondary reaction of the plurality of invasive cleavage assays in the multiplex. In some examples, the software then compares the amount of signal measured, or alternatively the rate of signal accumulation, to determine, by comparison with a threshold value, whether a target nucleic acid was detected.

Preferably, when the computer used for executing and/or analyzing a multiplex invasive cleavage assay is an integral component of an apparatus for performing and analyzing real-time nucleic acid amplification reactions, the apparatus preferably comprises a temperature-controlled incubator, a detection device for collecting signals, an analyzing device (*e.g.,* a computer or processor) for analyzing signals and an output device for displaying data obtained or generated by the analyzing device. The analyzing device may be connected to the temperature-controlled incubator through an input device known in the art, and/or connected to an output device known in the art for data display. In some examples, the temperature-controlled incubator is capable of temperature cycling.

As discussed below, the various components of an apparatus useful in connection with the disclosed methods will be conventional components familiar to those having an ordinary level of skill in the art. The temperature-controlled incubator may be of a conventional design which can hold a plurality of reaction tubes, or reaction samples in a temperature-controlled block in standard amplification reaction tubes or in wells of a multiwell plate. In one aspect, the detection system is suitable for detecting optical signals from one or more fluorescent labels. The output of the detection system (*e.g*., signals corresponding to those generated during a secondary reaction) can be fed to the computer for data storage and manipulation. In one example, the system detects multiple different types of optical signals, such as multiple different types of fluorescent labels and has the capabilities of a microplate fluorescence reader. The detection system is preferably a multiplexed fluorometer containing an excitation light source, which may be a visible light laser or an ultraviolet lamp or a halogen lamp, a multiplexer device for distributing the excitation light to the individual reaction tubes and for receiving fluorescent light from the reaction tubes, a filtering means for separating the fluorescence light from the excitation light by their wavelengths, and a detection means for measuring the fluorescence light intensity. Preferably, the detection system of the temperature-controlled incubator provides a broad detection range that allows flexibility of fluorophore choice, high sensitivity and excellent signal-to-noise ratio. Optical signals received by the detection system are generally converted into signals which can be operated on by the processor to provide data which can be viewed by a user on a display of a user device in communication with the processor. The user device may comprise a user interface or may be a conventional commercially available computer system with a keyboard and video monitor. Examples of data which can be displayed by the user device include amplification plots, scatter plots, sample value screens for all the tubes or reaction vessels in the assembly and for all labels used, an optical signal intensity screen (*e.g*., fluorescent signal intensity screen), a velocity screen (*e.g*., showing change in fluorescent intensity as a function of time), final call results, text reports, and the like.

Included within the scope of the disclosure are software-based products (*e.g*., software for instructing a computer to execute various procedural steps) for performing the data processing method. These include non-transitory (*e.g.*, software instructions stored on computer-readable media, such as magnetic media, optical media, "flash" memory devices, and computer networks. As well, the disclosure embraces a system or an apparatus that amplifies nucleic acids, detects nucleic acid amplification products, and processes results to indicate a quantitative result for target in a test sample. Although the various components of the apparatus preferably function in a cooperative fashion, there is no requirement for the components to be part of an integrated assembly (*e.g*., on a single chassis). However, in one example, components of the apparatus are connected together. Included within the meaning of "connected" are connections via wired and wireless connections.

Particularly falling within the scope of the disclosure is an apparatus or system that includes a computer linked to a device that amplifies nucleic acids and monitors amplicon synthesis as a function of cycle number or time, where the computer is programmed to execute analysis of accumulated fluorescence as disclosed herein. An exemplary system in accordance with the disclosure will include a temperature-controlled incubator, and a fluorometer capable of monitoring and distinguishing at least two, more preferably three, and still more preferably four or more wavelengths of fluorescent emissions. These emissions may be used to indicate target amplicon synthesis, and further used to extend the number of multiplex invasive cleavage assays that may be performed at one time.

### Data Processing Software

Software loaded and implemented in a computer that processes data from multiplex cleavage assays typically will instruct an early step that involves "collecting" certain data. This can involve retrieving quantitative information from a data file, and ensuring that the information is properly entered into an electronic spreadsheet that works in conjunction with the software. If the software is loaded into a computer component of an analytical system equipped with a fluorometer that monitors fluorescent signals in a reaction mixture undergoing testing or processing (*e.g*., as a function of time), then the data may be collected from the fluorometer of the instrument, either directly or indirectly (*e.g*., via a machine interface). The step of collecting data also embraces certain processing steps, for example calculating the slope of a line, calculating a rate of change, *etc.* The result of such processing steps can be considered part of the collected data to ensure relevant information is available for subsequent operations directed by the software.

Generally speaking, the collected data will relate to the multiplex methods disclosed herein. More particularly, the data will include information regarding magnitudes of fluorescent signals produced in a reaction mixture by a single fluorophore, where the secondary reactions of a plurality of different invasive cleavage assays contributed to the overall fluorescence reading. In some instances, results from a "negative control" reaction *(i.e.,* a reaction not including the analyte nucleic acid(s)) will be included among the collected data. "Background" signals also will be collected, generally reflecting fluorescent signal magnitudes before a particular reaction has begun. There can be a plurality of background fluorescence readings in a single procedure. Background readings indicate the detectable level of fluorescence before a particular secondary reaction has begun to generate significant increases of the fluorescent signal. This may be determined from a fluorescence reading taken before a reaction has begun, but also can be determined immediately after the reaction has begun and not had sufficient time to produce enough signal to observe meaningful changes in fluorescence.

Another feature of the software involves "comparing" different elements from the collected data sets prior to making a determination about the presence or amount of different target nucleic acids in a sample undergoing testing. The comparing step can involve simply determining whether the difference between two numerical values is above or below a threshold used for assigning significance. For example, a threshold value can be used for determining whether or not a determined difference between fluorescence readings of a test sample and a control sample is significant. Those having an ordinary level of skill in the art will appreciate that setting of thresholds for this purpose can involve use of positive controls (e.g., processed samples known to contain a target nucleic acid of interest), and generally involves only routine efforts. Whether or not a difference rises to the level of significance is sometimes expressed by use the terms "substantially greater than" *(i.e.,* a significant difference) or "substantially the same as" (*e.g*., essentially equal).

Software instructions also direct the making of certain "determinations," and then "outputting" a record of those determinations. Determinations can rely on checking a standard look-up table, which can be stored on the computer or provided with the software. Examples of entries in a look-up table would include assigning a determination about the presence or absence of a target nucleic acid based on whether or not certain criteria were met. Whether or not background signals were substantially the same or different; and whether or not time- or cycle-dependent rates of change (*e.g*., judged by the slope of a line) exceeds or does not exceed a control value are examples of such criteria. Outputted determinations can take the form of tangible (*e.g*., non-transitory) outputs, such as records printed on paper. Alternatively, outputted determinations may take the form of information presented on an electronic display or computer monitor.

Some preferred examples of software useful in connection with multiplex invasive cleavage assays find particular application to processing of real-time nucleic acid amplification results, either alone or in combination with endpoint results.

Again, software instructions for these applications will direct an early step that involves collecting certain data concerning the multiplex invasive cleavage assays. Typically this data relates to background readings for fluorescent signals measured before particular secondary reactions have substantially begun to produce increases in the fluorescent signal. As well, there will be at least one fluorescent signal measured after the particular secondary reaction has begun. Whether or not the reaction is active or inactive at a particular time can be controlled by adjusting the reaction temperature to correspond to the temperature optimum for the secondary reaction, or to be different by at least 5°C (at which point cycling in the reaction is substantially reduced). When amplification of more than a single target nucleic acid is being monitored in the multiplex invasive cleavage assay using the real-time format, the software can essentially assign background status to the final fluorescent signal read for a prior reading associated with a different secondary reaction, and associates any increase beyond that background level with the new secondary reaction being monitored. The software tracks progressive increases in fluorescence emission from a reaction mixture, and assigns incremental increases to one or another of the plurality of secondary reactions, as appropriate.

In the case where a real-time formatted assay *(i.e.,* a multiplex invasive cleavage assay being applied to detection of an amplified target nucleic acid as the reaction cycles are occurring) is followed by an endpoint determination in the multiplex invasive cleavage assay, the magnitude of the fluorescent signal attributed to the target nucleic acid being monitored in the endpoint portion of the assay is calculated by the software as the difference between the endpoint fluorescence signal reading and the "background," which corresponds to the final fluorescence reading of the real-time monitored portion of the assay.

### EXAMPLES

The following Example demonstrates how primary reactions for two multiplexed invasive cleavage assays (*i.e.,* one being specific for a human target nucleic acid, the other specific for a bacterial target nucleic acid), could be carried out under a single temperature condition, while secondary reactions took place under different temperature conditions. Fluorescent signals were monitored as a function of time during the linear secondary reaction conducted at the lower of two temperature conditions. Only one of the two multiplexed secondary reactions was active under each of the two temperature conditions at any time. This confirmed that the multiplexed secondary reactions had been temporally isolated.

Example 1 describes procedures and results that demonstrated multiplex invasive cleavage assays used for detecting multiple targets in a single reaction mixture that included a single fluorescent reporter. Results confirmed that a plurality of target nucleic acids were detected independently.

### Example 1

### Detection of Human and Bacterial Target Nucleic Acids in a Multiplex Invasive Cleavage Assay

Complete reaction mixtures for detecting rRNA from various biological samples were prepared using the Panther™ system (Hologic, Inc.) for automated *in vitro* diagnostic testing. Reaction mixtures included oligonucleotide primers and reagents (*e.g*., four deoxyribonucleotides, buffers, salts, *etc*.) needed for synthesizing a first strand cDNA complementary to human or Prevotella bacterial rRNA that may be present in a test sample. Reaction mixtures further included for each of the two targets: one invasive probe; one primary probe that included a 5' flap sequence; and one FRET cassette labeled with the combination of a fluorescein (FAM) fluorophore and Eclipse quencher (Epoch Biosciences, Inc.). Thus, each of the FRET cassettes included the same interactive label pair in energy transfer relationship, where fluorescence emission was quenched when the fluorescein moiety was attached to the same FRET cassette as the quencher. The Tms for hybridization of the human target cDNA with the human-specific invasive probe and primary probe were 69°C and 64°C, respectively. The Tm for hybridization of the 5' flap cleaved from the human-specific primary probe to the FRET cassette was 63°C. The Tms for hybridization of the bacterial target cDNA with the bacterial-specific invasive probe and primary probe were in the range of from about 68°C to 70°C (*e.g.,* 69°C) and from about 63°C to 65°C (*e.g.,* 64°C), respectively. The Tm for hybridization of the 5' flap cleaved from the bacterial-specific primary probe to the FRET cassette was 43°C.

FIG. 7 illustrates a sample work flow diagram for conducting invasive cleavage assays, including multiplex invasive cleavage assays, on the Panther™ system configured for real-time fluorescence monitoring. Rectangles represent time periods at different stations and temperatures in the instrument. Components of the reaction mixture were dispensed in two aliquots into a single reaction vessel containing the test sample. Addition of reagents took place in the AmpLoad station, where robotic pipetting delivered reagents into the reaction vessel. Oligonucleotides *(i.e.,* reverse transcriptase primers, invasive probes, primary probes, and FRET cassettes) and reagents (buffer, salts, dNTPs) were delivered in one aliquot, while MMLV reverse transcriptase and thermostable Cleavase® X enzyme (Hologic, Inc.) were delivered in a second aliquot. Once reaction mixtures were complete, no additional reagents were added to the reaction vessel until all results had been obtained. A trial using specimen transport medium that did not contain either human or bacterial DNA served as a negative control. Likewise, human and bacterial cell lysates containing rRNAs served as positive controls for the presence of human and bacterial nucleic acids, respectively.

Reaction mixtures were processed on the automated Panther™ instrument essentially according to the workflow diagram in FIG. 7. Reaction mixtures were incubated for about 5 minutes at 44°C to permit synthesis of a cDNA strand from each of the two *(i.e.,* human and bacterial) target sequences by the process of reverse transcription. Reaction vessels containing the mixtures were then transported to a 64°C incubator for about 26 minutes. No fluorescence readings were taken during this high temperature incubation. The primary and secondary invasive cleavage reactions for the human-specific assay, and the primary invasive cleavage reaction (but not the secondary invasive cleavage reaction) for the Prevotella-specific assay took place during the 64°C incubation. Next, reaction vessels containing the mixtures were transported to a 43°C incubator where only the secondary invasive cleavage reaction of the Prevotella-specific assay took place. Neither invasive cleavage reaction of the human-specific assay was active during the 43°C incubation. The primary invasive cleavage reaction of the Prevotella-specific assay also was not active during the 43°C incubation. A period of about 1 minute elapsed before fluorescent monitoring as a function of time commenced under the 43°C temperature condition. Fluorescent signals were monitored for about 50 minutes at 43°C using a single channel of the fluorometer on the automated Panther™ instrument. More particularly, emission from fluorescein was monitored as an indicator of reaction progress.

FIG. 8A displays raw fluorescence as a function of reaction time during the 43°C incubation. The figure presents combined results obtained for a negative control sample (lower line); a sample that included the Prevotella bacterial target nucleic acid, but not the human target nucleic acid (middle line); and a sample that included both the Prevotella bacterial target and the human target nucleic acid (upper line). The negative control indicated a low level baseline activity, where fluorescence readings increased slightly with time, as expected. The reaction mixture that included the Prevotella bacterial target but not the human target nucleic acid initiated from substantially the same level of starting fluorescence as the negative control, and then increased steadily with time. This indicated the presence of the Prevotella target, and further indicated that the secondary reaction specific for the human target nucleic acid made no contribution to the measured fluorescence signal. Stated differently, the invasive cleavage assay specific for the human target nucleic acid, while present during the 64°C incubation with a sample that did not include the human target nucleic acid, did not generate any detectable signal. The result obtained for the trial that included both human and bacterial target nucleic acids initiated from a higher starting level of fluorescence because the secondary reaction for detection of the human target was already complete at the time fluorescence was read under the 43°C temperature condition. The higher starting fluorescence reading indicated the presence of human target nucleic acid in the sample undergoing testing. The further time-dependent increase in fluorescence indicated the bacterial target nucleic acid also was present in the sample being tested. Moreover, the substantially similar slopes of the middle and upper lines on the plot indicated that only the secondary reaction specific for the bacterial target nucleic acid was active when fluorescence was read during the 43°C incubation. This again evidenced temporal isolation of the two secondary reactions in the multiplex assay, even though the same fluorescent reporter was used for detection of both target nucleic acids.

FIG. 8B presents data derived from results similar to those shown in FIG. 8A. The minimum fluorescence signal (y-axis) measured at the start of the 43°C incubation is shown for a variety of samples. These included samples having high levels of human target, but low levels of bacterial target nucleic acid (186E2 and 191L_2); a sample having low levels of both human and bacterial target nucleic acids (BV-10_2); a sample having low levels of human target, but high levels of bacterial target nucleic acid (CS_P_Gvag_2); a sample having high levels of both human and bacterial target nucleic acids (CS_P_Gvag_H_2); a negative control that did not include either human or bacterial target nucleic acid (STM); and two positive control samples that included high levels of human target, but low levels of bacterial target nucleic acid (TP-07_2 and TP-08_2). The magnitude of the minimum fluorescence at the start of the 43°C incubation indicated whether or not the sample undergoing testing included the human target nucleic acid. While this determination could have been made by monitoring appearance of the fluorescent signal during the 64°C incubation (*i.e.,* at a time when the primary and secondary invasive cleavage reactions specific for the human target were active), the approach described here showed that was unnecessary. Instead, the determination could be made using an end-point measurement.

FIG. 8C also presents data derived from results similar to those shown in FIG. 8A. In this instance, velocity of the accumulated fluorescent signal was determined from the slopes of lines similar to those in FIG. 8A. Velocity measurements were useful for determining whether or not different test samples included the bacterial target nucleic acid. It should be apparent that the upper and middle lines shown in FIG. 8A were characterized by positive slopes substantially greater than the slope of the negative control line. Similarly, two of the samples shown in FIG. 8C exhibited velocities substantially in excess of the negative control, thereby indicating these samples included the bacterial target nucleic acid.

The following Example illustrates multiplex PCR amplification of a combined three target nucleic acids encoding different proteins (*i.e.,* Factor II, Factor V, and MTHFR 1298) of the human blood clotting cascade. Each of the three amplified targets contained either a wild-type or mutant sequence distinguished by a SNP. The different amplified targets were detected using multiplex invasive cleavage assays specific for either the wild-type or mutant sequence. Since the human is a diploid organism, and since three genetic targets were amplified, nine possible genotypes could be detected *(i.e.,* homozygous wild-type/homozygous mutant/heterozygote). To illustrate the combination of endpoint detection and real-time monitoring in a single multiplex invasive cleavage assay using a single fluorophore and detection channel for three amplified targets, and a different fluorophore and detection channel for the remaining three targets. By this approach, for each amplified target it was possible to identify which of two different SNPs was present.

Example 2 describes procedures that demonstrated how a multiplex invasive cleavage assay employing a single fluorophore could be used in both real-time and endpoint formats to permit independent detection of target nucleic acids. In this instance, a single fluorophore was used for detecting three different target nucleic acids in a reaction mixture. A second fluorophore was used for detecting three different sequences that were distinguished by SNPs, also using the multiplex format.

### Example 2

### Integrated Multiplex PCR with Multiplex Invasive Cleavage Assay Using Both Real-Time Monitoring and Endpoint Detection

A single reaction mixture was prepared for use on an Mx3005P real-time PCR instrument (Stratagene). The reaction mixture contained independent sets of paired PCR primers for amplifying SNP-containing segments of the following three human gene sequences: Factor II, Factor V, and MTHFR 1298. As well, the reaction mixture included invasive probes directed to a highly conserved sequence in amplification products for each of the three target genes, together with paired sets of primary probes (one primary probe of each set being specific for a wild-type sequence; the other being specific for a mutant SNP sequence in the same amplified gene). Accordingly, the reaction mixture contained six primary probes, each having a different 5' flap sequence. There also were three paired sets of FRET cassettes, one for each primary probe, where a 5' flap cleaved from one of the primary probes could hybridize to a complementary sequence found in only one of the FRET cassettes. FRET cassettes labeled with Redmond Red dye and Eclipse quencher were used for invasive cleavage assays that detected wild-type Factor II and Factor V, and mutant MTHFR 1298. Fluorescent emission signals generated by cleavage of these FRET cassettes were detected in the ROX channel of the PCR instrument. FRET cassettes labeled with FAM and either Eclipse quencher or BlackBerry® Quencher BBQ-650®-dT were used for invasive cleavage assays that detected mutant Factor II and Factor V, and wild-type MTHFR 1298. More specifically, FRET cassettes for detecting cleaved 5'-flaps indicating the presence of Factor II and MTHFR 1298 target nucleic acids harbored the BBQ-650®-dT Quencher, while the FRET cassette for detecting the cleaved 5'-flap indicating the presence of Factor V target nucleic acid harbored the Eclipse quencher. Fluorescent emission signals generated by cleavage of these FRET cassettes were detected in the FAM channel of the PCR instrument. The arrangement of oligonucleotides is essentially schematized in FIG. 6, except that oligonucleotides for amplifying and detecting the MTHFR 677 target nucleic acid were omitted. Finally, thermostable Taq DNA polymerase and Cleavase® 2.0 enzymes, in addition to deoxyribonucleotide triphosphates, pH buffer, and salts were included to complete the reaction mixtures.

Thermal cycling and fluorescence detection and monitoring was conducted essentially as follows. The reaction mixture was initialized for 30 seconds at 25°C. A single incubation at 95°C for 2 minutes denatured nucleic acids at the start of the procedure. This was followed by 40 cycles of 94°C for 5 seconds, and 72°C for 20 seconds. Primer extension and detection of the fluorescent signal resulting from the primary and secondary invasive cleavage reactions for detecting Factor II amplified nucleic acids both took place during the 72°C incubation steps. As well, the primary invasive cleavage reaction, but not the secondary invasive cleavage reaction, for detecting Factor V also took place during the 72°C incubation steps. This was followed by a 5 minute incubation at 99°C to substantially inhibit further activity of the Taq DNA polymerase enzyme. Next, there were 9 cycles of 20 seconds at 58°C (*i.e.,* for a 3 minute step) to carry out and monitor fluorescent signals generated in the secondary invasive cleavage reaction, but not the primary invasive cleavage reaction, of the invasive cleavage assay for detecting Factor V. This was followed by 12 cycles of 20 seconds at 43°C *(i.e.,* for a 4 minute step), during which time the primary and secondary invasive cleavage reactions of the invasive cleavage assay specific for the MTHFR 1298 target nucleic acid took place, and during which time fluorescent signals were measured. In this way at least the secondary reactions for the different invasive cleavage assays were separated in time, and fluorescent signals generated thereby were attributed to one of the multiplexed assays at each cycle or step of the procedure. More specifically, the magnitudes of fluorescence signals read during the various intervals were processed so that signals could be attributed to a secondary reactions corresponding to one of the six target nucleic acids to be detected. This essentially involved subtraction of background fluorescent signals that preceded the secondary reaction being assessed. For example, the magnitude of a fluorescent signal attributed to a secondary reaction for detecting Factor V at 58°C could be determined by subtracting from each 58°C fluorescence reading the magnitude of the fluorescent signal determined after the last reading at 72°C for the Factor II signal, or alternatively the first fluorescent signal read at 58°C.

Results indicated the three different wild-type target nucleic acid sequences could each be detected independently using FRET cassettes harboring one fluorophore, while three SNPs could each be detected independently using FRET cassettes harboring a different fluorophore. FIG. 9A presents raw fluorescence readings as a function of reaction cycle number. FIG. 9B presents the data from FIG. 9A following background subtraction to isolate the fluorescence increase due to detection of one of the three multiplexed invasive cleavage assays employing a particular fluorophore. More specifically, the magnitude of the initial fluorescence reading measured at the 72°C incubation temperature used for detecting fluorescence generated in the secondary reaction of the invasive cleavage reaction for detecting Factor II was subtracted from all subsequent fluorescence readings taken at 72°C. This was done for the data set collected for ROX channel signals (representing WT Factor II detection), and separately for FAM channel signals (representing detection of the Factor II SNP sequence). Similarly, the first fluorescence reading from the 58°C incubation was used as the background fluorescence for incubations conducted at 58°C. The values were subtracted from all subsequent fluorescence readings taken at 58°C to normalize the data sets for detection of Factor V WT and Mut sequences. Finally, the first fluorescence reading from the data set collected during the 43°C incubation was used as the background value that was subtracted from fluorescence values read during the 44°C incubation (MTHFR 1298). The figure thus presents background-subtracted fluorescence readings for each of the FAM and ROX channels, and illustrates how each of the two fluorescent labels was used for detecting and distinguishing three different target nucleic acid sequences.

Example 3 generally describes procedures that can be used for establishing the presence and concentration of a target nucleic acid relative to a single calibration standard.

### Example 3

### Monitoring of a Plurality of Multiplexed Invasive Cleavage Assays Independently in a Time-Dependent Fashion

A reaction mixture is prepared containing both a sample to be tested for an analyte target nucleic acid, and a known amount of a first calibration standard. The known amount of the first calibration standard used in this Example is 1,000 copies/ml. The reaction mixture is disposed in a single tube, and placed into the block of a thermal cycling instrument equipped with a fluorometer and connected to a computer. The computer controls the thermal cycling profile and records fluorescence readings for specified periods of time during specified temperature intervals. The first calibration standard comprises a nucleic acid sequence substantially similar to the portion of the analyte target nucleic acid that is to be amplified, except for nucleotide differences that can be distinguished by the use of different invasive cleavage assays. A first invasive cleavage assay that detects the amplified first calibration standard, but not the amplified analyte target nucleic acid, comprises an invasive probe, a first primary probe that includes a first 5' flap, and a first FRET cassette that includes a fluorescein moiety and an appropriate quencher moiety, such as the Eclipse quencher. A second invasive cleavage assay that detects the amplified analyte target nucleic acid, but not the amplified first calibration standard, comprises the same invasive probe, a second primary probe that includes a second 5' flap, and a second FRET cassette that includes a fluorescein moiety and appropriate quencher moiety, such as the Eclipse quencher. The reaction mixture further includes a thermostable DNA polymerase and the Cleavase® enzyme (Hologic, Inc.). The temperature optima for the different secondary reactions of the invasive cleavage assays differ by at least 10°C. The primary and secondary reactions of the first invasive cleavage assay have a temperature optimum of 72°C. The second invasive cleavage assay employs a primary reaction having a temperature optimum of 72°C, and secondary reaction having a temperature optimum of 58°C. The analyte target nucleic acid and the first calibration standard are co-amplified in the reaction mixture using the thermostable DNA polymerase and a substantially similar set of DNA primers. Thermal cycling conditions include 40 cycles of: 94°C for 5 seconds to denature nucleic acids; 72°C for 20 seconds; and 58°C for 20 seconds. The 72°C step permits synthesis of all nucleic acid amplification products; permits all primary invasive cleavage reactions to take place; and permits the secondary reaction specific for the first calibration standard (but not the secondary reaction specific for the analyte target nucleic acid) to take place. The 58°C step permits the secondary reaction specific for the analyte target nucleic acid (but not the secondary reaction specific for the first calibration standard) to take place. Fluorescence readings for the fluorescein emission wavelength are taken during the 72°C and 58°C temperature cycles using the fluorometer of the PCR instrument. The computer receives the fluorescence data inputs and processes the information.

During each cycle of the thermal cycling profile, the magnitude of the fluorescent increase during the 72°C temperature step is attributed to an increase in the first calibration standard amplicon production, and the magnitude of the fluorescent increase during the 58°C temperature step is attributed to an increase in production of the analyte target nucleic acid amplicon. Different sigmoid plots of the resulting fluorescence as a function of cycle number are established following background subtraction, and the point at which each run curve crosses a predetermined fluorescence threshold value is determined. Both assays need not have the same threshold. The run curve representing the first calibration standard crosses the threshold at cycle number 25 which is within a window of "normal performance" (*e.g*., 24-26 cycles), establishing the Ct value of 25. The window of "normal performance" is established during assay development and indicates that the sample/control extraction was suitably efficient *(i.e.,* high enough to provide the expected amount of control template) and that the reaction is free of detectable inhibitors which could impact performance. The run curve representing amplification of the analyte target nucleic acid crosses the threshold at cycle number 22. According to one interpretation of the data, the computer makes the cycle threshold determinations and outputs a result indicating that the sample being tested contains the analyte target nucleic acid in an amount greater than 1,000 copies/ml. If the Ct value determined for the sigmoid plot of the analyte target nucleic acid had a value greater than 25, then the computer would instead generate an output that indicates the sample being tested contains the analyte target nucleic acid in an amount less than 1,000 copies/ml. According to an alternative interpretation of the data, the computer makes the cycle threshold determinations and outputs a result indicating that the sample being tested contains the analyte target nucleic acid at 10,000 copies/ml based on known amplification efficiencies (3 cycles equates to about 10 fold difference in concentration) between the internal calibrator and the target analyte when the Ct value determined for the internal calibrator falls within the "normal range." If the Ct value determined for the sigmoid plot of the analyte target nucleic acid has a value of 31, the computer would generate an output that indicates the sample being tested contains the analyte target nuclei acid at a concentration of about 10 copies/ml.

Example 4 further illustrates how multiplex invasive cleavage assays can be used for quantifying a target nucleic acid.

### Example 4

### Use of a Plurality of Multiplexed Invasive Cleavage Assays to Quantify an Analyte Target Nucleic Acid

The procedure described under Example 3 is modified to include a second calibration standard at 10,000 copies/ml. The second calibration standard is amplified using the same primer set that amplifies the first calibration standard and the analyte target nucleic acid. The structure of the second calibration standard is substantially identical to the structure of the first calibration standard, except that one or more nucleotides at the position that distinguishes the first calibration standard from the analyte target nucleic acid is unique *(i.e.,* being different from the nucleotide at that position in each of the first calibration standard and the analyte target nucleic acid). A third invasive cleavage assay in the multiplex reaction mixture is used to detect the second calibration standard, but not the analyte target nucleic acid or the first calibration standard. The third invasive cleavage assay comprises the invasive probe, a third primary probe that includes a third 5' flap, and a third FRET cassette that includes a fluorescein moiety and an appropriate quencher moiety, such as the Eclipse quencher. The temperature optimum for the primary and secondary reactions of the invasive cleavage assay that detects the second calibration standard is 43°C, and so differs from the temperature optima of the other invasive cleavage assays by at least 10°C. The analyte target nucleic acid, the first calibration standard, and the second calibration standard are co-amplified in the reaction mixture using the same set of DNA primers. Thermal cycling conditions include 40 cycles of: 94°C for 5 seconds to denature nucleic acids; 72°C for 20 seconds; 58°C for 20 seconds; and 43°C for 20 seconds. The 72°C step permits synthesis of all nucleic acid amplification products; permits all primary invasive cleavage reactions to take place; and permits the secondary reaction specific for the first calibration standard (but not the secondary reactions specific for either the analyte target nucleic acid, or the second calibration standard) to take place. The 58°C step permits the secondary reaction specific for the analyte target nucleic acid (but not the secondary reaction specific for either the first calibration standard, or the second calibration standard) to take place. The 43°C step permits the secondary reaction specific for the second calibration standard (but not the secondary reaction specific for either the first calibration standard, or the analyte target nucleic acid) to take place. Fluorescence readings for the fluorescein emission wavelength are taken during the 72°C, 58°C, and 43°C temperature cycles using the fluorometer of the PCR instrument. The computer receives the fluorescence data inputs and processes the information.

During each cycle of the thermal cycling profile, the magnitude of the fluorescence increase during the 72°C temperature step is attributed to an increase in first calibration standard amplicon production, the magnitude of the fluorescence increase during the 58°C temperature step is attributed to an increase in analyte target nucleic acid amplicon production, and the magnitude of the fluorescence increase during the 43°C temperature step is attributed to an increase in second calibration standard amplicon production. Different sigmoid plots of fluorescence as a function of cycle number are established following background fluorescence subtraction, and the point at which each run curve crosses a predetermined fluorescence threshold value of 2,000 RFU is determined. The run curve representing amplification of the first calibration standard crosses the threshold at cycle number 25, establishing a Ct value of 25. The run curve representing amplification of the second calibration standard crosses the threshold at cycle number 20, establishing a Ct value of 20. The run curve representing amplification of the analyte target nucleic acid crosses the threshold at cycle number 22, establishing a Ct value of 22. The computer makes these determinations and outputs a result indicating that the sample being tested contains the analyte target nucleic acid in an amount between 1,000 copies/ml and 10,000 copies/ml. Alternatively, the computer establishes a calibration curve or line using the Ct values determined for the two calibration standards (*i.e.,* 20 and 25), and then uses the calibration curve or line to determine the quantity of analyte target nucleic acid from the determined Ct value. For example, a printed in communication with the computer outputs a printed paper record indicating the analyte target nucleic acid is present at a level of 1 x 10e3.6 (*i.e.,* about 3,980) copies/ml.

## Claims

1. A method of determining which of a plurality of different target nucleic acids are present in a test sample, the method comprising the steps of:
(a) in a reaction mixture containing nucleic acids from the test sample, serially permitting, in order, first and second secondary reactions of a multiplex invasive cleavage assay to take place, each of the secondary reactions being active under a different temperature condition, and each secondary reaction being specific for only one of the target nucleic acids;
(b) monitoring the reaction mixture for production of a fluorescent signal only as the second of the two secondary reactions is taking place at the lower of two temperature conditions, each of the different temperature conditions being permissive for only one of the secondary reactions, and each of the secondary reactions producing the same fluorescent signal; and
(c) determining which of the target nucleic acids is present in the test sample by comparing a magnitude of the fluorescent signal produced in the reaction mixture during step (b) with a threshold value or threshold criterion.

2. The method of claim 1, wherein the test sample comprises products of a nucleic acid amplification reaction, and wherein each of the plurality of target nucleic acids is a product of the nucleic acid amplification reaction; or
wherein the test sample comprises products of a multiplex nucleic acid amplification reaction, and wherein each of the plurality of target nucleic acids is a product of the multiplex nucleic acid amplification reaction.

3. The method of any one of the preceding claims, wherein the reaction mixture comprises a thermostable DNA polymerase enzyme; and/or
wherein each of the different temperature conditions differs from the others by 5°C to 30°C; preferably,
wherein each of the different temperature conditions differs from the others by 5°C to 15°C.

4. The method of any of the preceding claims, wherein step (c) comprises determining which of the target nucleic acids is present in the test sample based on the amount of the fluorescent signal produced in the reaction mixture during step (b); or
wherein step (c) comprises determining which of the target nucleic acids is present in the test sample based on the rate of production of the fluorescent signal in the reaction mixture during step (b).

5. A method of determining whether a test sample contains one or both of a first target nucleic acid and a second target nucleic acid, the method comprising the steps of:
(a) preparing a reaction mixture by combining each of,
(i) the test sample,
(ii) a first set of oligonucleotides for conducting a first invasive cleavage assay specific for detection of the first target nucleic acid, the first invasive cleavage assay comprising a primary reaction and a secondary reaction, and the first set of oligonucleotides comprising,
a first invasive probe specific for the first target nucleic acid,
a first primary probe specific for the first target nucleic acid, and
a first FRET cassette,
(iii) a second set of oligonucleotides for conducting a second invasive cleavage assay specific for detection of the second target nucleic acid, the second invasive cleavage assay comprising a primary reaction and a secondary reaction, the second set of oligonucleotides comprising,
a second invasive probe specific for the second target nucleic acid,
a second primary probe specific for the second target nucleic acid, and
a second FRET cassette,
wherein each of the first and second FRET cassettes comprises a donor moiety and an acceptor moiety in energy transfer relationship such that emission from the donor moiety is quenched when the donor moiety and the acceptor moiety are both attached to the same FRET cassette, and
wherein the donor moiety of the first FRET cassette is identical to the donor moiety of the second FRET cassette, and
(iv) a flap endonuclease (FEN) enzyme;
(b) incubating the reaction mixture for a first period of time at a first temperature, the first temperature being permissive for the primary reaction of the first and second invasive cleavage assays, and for the secondary reaction of the first invasive cleavage assay, whereby a first signal associated with the donor moiety of the first FRET cassette is generated if the test sample includes the first target nucleic acid;
(c) measuring any of the first signal that may have been generated during step (b) as an indication of the presence of the first target nucleic acid in the test sample;
(d) incubating the reaction mixture for a second period of time at a second temperature, the second temperature being permissive for the secondary reaction of the second invasive cleavage assay, whereby a second signal associated with the donor moiety of the second FRET cassette is generated if the test sample includes the second target nucleic acid;
(e) measuring any of the second signal that may have been generated during step (d) as an indication of the presence of the second target nucleic acid in the test sample; and
(f) determining from the first signal measured in step (c) whether the sample contains the first target nucleic acid, and from the second signal measured in step (e) whether the sample contains the second target nucleic acid,
wherein the secondary reaction of the first invasive cleavage assay does not substantially generate any signal greater than a background signal at the second temperature in step (d), and
wherein the secondary reaction of the second invasive cleavage assay does not substantially generate any signal greater than a background signal at the first temperature in step (b).

6. The method of claim 5, wherein the donor moiety is a fluorophore.

7. The method of claim 5, wherein the first and second temperatures in the incubating steps differ by 5-30°C.

8. The method of claim 7, wherein the first and second temperatures in the incubating steps differ by 5-15°C.

9. The method of claim 5 or 7, wherein step (e) comprises measuring over time any of the second signal that may have been generated during step (d), whereby a rate of change of the signal generated in step (d) is established.

10. The method of claim 5, wherein step (c) comprises identifying the magnitude of the first signal that may have been generated during step (b), and
wherein step (f) further comprises determining that the first target nucleic acid is present in the sample if the identified magnitude exceeds a magnitude threshold for detecting the first target nucleic acid; or
wherein step (c) comprises identifying the magnitude of the first signal that may have been generated during step (b), and
wherein step (f) further comprises determining that the first target nucleic acid is not present in the sample if the identified magnitude does not exceed a magnitude threshold for detecting the first target nucleic acid; or
wherein step (e) comprises measuring over time any of the second signal that may have been generated during step (d), whereby a rate of change of the signal generated in step (d) is established, and
wherein step (f) further comprises determining that the second target nucleic acid is present in the sample if the rate of change exceeds a velocity threshold for detecting the second target nucleic acid; or
wherein step (e) comprises measuring over time any of the second signal that may have been generated during step (d), whereby a rate of change of the signal generated in step (d) is established, and wherein step (f) further comprises determining that the second target nucleic acid is not present in the sample if the rate of change does not exceed a velocity threshold for detecting the second target nucleic acid.

11. The method of claim 9, wherein the rate of change is a time-dependent rate of change; or
wherein the rate of change is a cycle-dependent rate of change; or
wherein the threshold for detecting the second target is a threshold velocity.

12. The method of any one of claims 5 to 11, wherein the first temperature of step (b) is greater than the second temperature of step (d); and/or
wherein the threshold for detecting the first target nucleic acid in step (f) is a threshold magnitude; and/or
wherein steps (b)-(e) are repeated in sequence a plurality of times before performing step (f).

## Patentansprüche

1. Verfahren zum Bestimmen, welche einer Vielzahl unterschiedlicher Zielnukleinsäuren in einer Testprobe vorhanden ist, wobei das Verfahren die folgenden Schritte umfasst:
a) der Reihe nach seriell erfolgendes Erlauben, in einer Nukleinsäuren aus der Testprobe enthaltenden Reaktionsmischung, dass erste und zweite sekundäre Reaktionen eines Multiplex-Assays zur invasiven Spaltung stattfinden, wobei jede der sekundären Reaktionen unter einer unterschiedlichen Temperaturbedingung aktiv ist und jede sekundäre Reaktion nur für eine der Zielnukleinsäuren spezifisch ist;
b) Überwachen der Reaktionsmischung bezüglich der Erzeugung eines Fluoreszenzsignals, wenn nur die zweite der zwei sekundären Reaktionen bei der unteren der zwei Temperaturbedingungen stattfindet, wobei jede der unterschiedlichen Temperaturbedingungen nur eine der sekundären Reaktionen erlaubt und jede der sekundären Reaktionen das gleiche Fluoreszenzsignal erzeugt; und
c) Bestimmen, welche der Zielnukleinsäuren in der Testprobe vorhanden ist, durch Vergleichen einer Größenordnung des Fluoreszenzsignals, das in der Reaktionsmischung während Schritt b) erzeugt worden ist, mit einem Schwellenwert oder einem Schwellenwertkriterium.

2. Verfahren nach Anspruch 1, wobei die Testprobe Produkte einer Nukleinsäureamplifikationsreaktion umfasst und wobei jede der Vielzahl von Zielnukleinsäuren ein Produkt der Nukleinsäureamplifikationsreaktion ist; oder
wobei die Testprobe Produkte einer Multiplex-Nukleinsäureamplifikationsreaktion umfasst und wobei jede der Vielzahl von Zielnukleinsäuren ein Produkt der Multiplex-Nukleinsäureamplifikationsreaktion ist.

3. Verfahren nach einem der vorstehenden Ansprüche, wobei die Reaktionsmischung ein thermostabiles DNA-Polymerase-Enzym umfasst; und/oder
wobei sich jede der unterschiedlichen Temperaturbedingungen von den anderen um 5 °C bis 30 °C unterscheidet;
wobei sich jede der unterschiedlichen Temperaturbedingungen von den anderen um vorzugsweise 5 °C bis 15 °C unterscheidet.

4. Verfahren nach einem der vorstehenden Ansprüche, wobei Schritt c) das Bestimmen, welche der Zielnukleinsäuren in der Testprobe vorhanden ist, basierend auf der Menge des in der Reaktionsmischung während Schritt b) erzeugten Fluoreszenzsignals umfasst; oder
wobei Schritt c) das Bestimmen, welche der Zielnukleinsäuren in der Testprobe vorhanden ist, basierend auf der Erzeugungsrate des Fluoreszenzsignals in der Reaktionsmischung während Schritt b) umfasst.

5. Verfahren zum Bestimmen, ob eine Testprobe eine oder beide einer ersten Zielnukleinsäure und einer zweiten Zielnukleinsäure enthält, wobei das Verfahren die folgenden Schritt umfasst:
a) Herstellen einer Reaktionsmischung, indem Folgendes kombiniert wird:
i) die Testprobe,
ii) eine erste Gruppe von Oligonukleotiden zum Durchführen eines ersten Assays zur invasiven Spaltung zum Nachweis der ersten Zielnukleinsäure, wobei das erste Assay zur invasiven Spaltung eine primäre Reaktion und eine sekundäre Reaktion umfasst und die erste Gruppe von Oligonukleotiden Folgendes umfasst:
eine erste invasive Sonde, die für die erste Zielnukleinsäure spezifisch ist;
eine erste primäre Sonde, die für die erste Zielnukleinsäure spezifisch ist, und
eine erste FRET-Kassette,
iii) eine zweite Gruppe von Oligonukleotiden zum Durchführen eines zweiten Assays zur invasiven Spaltung zum Nachweis der zweiten Zielnukleinsäure, wobei das zweite Assay zur invasiven Spaltung eine primäre Reaktion und eine sekundäre Reaktion umfasst und die zweite Gruppe von Oligonukleotiden Folgendes umfasst:
eine zweite invasive Sonde, die für die zweite Zielnukleinsäure spezifisch ist;
eine zweite primäre Sonde, die für die zweite Zielnukleinsäure spezifisch ist, und
eine zweite FRET-Kassette,
wobei jede der ersten und zweiten FRET-Kassette eine Donatormoleküleinheit und eine Akzeptormoleküleinheit in einer Energieübertragungsbeziehung umfasst, sodass die Emission von der Donatormoleküleinheit gelöscht wird, wenn die Donatormoleküleinheit und die Akzeptormoleküleinheit beide an derselben FRET-Kassette hängen, und
wobei die Donatormoleküleinheit der ersten FRET-Kassette mit der Donatormoleküleinheit der zweiten FRET-Kassette identisch ist, und
iv) ein Flap-Endonuklease(FEN)-Enzym;
b) Inkubieren der Reaktionsmischung über einen ersten Zeitraum bei einer ersten Temperatur, wobei die erste Temperatur die primäre Reaktion des ersten und zweiten Assays zur invasiven Spaltung und die sekundäre Reaktion des ersten Assays zur invasiven Spaltung erlaubt, wodurch ein mit der Donatormoleküleinheit der ersten FRET-Kassette assoziiertes erstes Signal erzeugt wird, wenn die Testprobe die erste Zielnukleinsäure einschließt;
c) Messen eines beliebigen des ersten Signals, das möglicherweise während Schritt b) erzeugt worden ist, als Hinweis auf das Vorhandensein der ersten Zielnukleinsäure in der Testprobe;
d) Inkubieren der Reaktionsmischung über einen zweiten Zeitraum bei einer zweiten Temperatur, wobei die zweite Temperatur die sekundäre Reaktion des zweiten Assays zur invasiven Spaltung erlaubt, wodurch ein mit der Donatormoleküleinheit der zweiten FRET-Kassette assoziiertes zweites Signal erzeugt wird, wenn die Testprobe die zweite Zielnukleinsäure einschließt;
e) Messen eines beliebigen des zweiten Signals, das möglicherweise während Schritt d) erzeugt worden ist, als Hinweis auf das Vorhandensein der zweiten Zielnukleinsäure in der Testprobe; und
f) Bestimmen aus dem in Schritt c) gemessenen ersten Signal, ob die Probe die erste Zielnukleinsäure enthält, und aus dem in Schritt e) gemessenen zweiten Signal, ob die Probe die zweite Zielnukleinsäure enthält,
wobei die sekundäre Reaktion des ersten Assays zur invasiven Spaltung bei der zweiten Temperatur in Schritt d) im Wesentlichen kein Signal erzeugt, das größer ist als ein Hintergrundsignal, und
wobei die sekundäre Reaktion des zweiten Assays zur invasiven Spaltung bei der ersten Temperatur in Schritt b) im Wesentlichen kein Signal erzeugt, das größer ist als ein Hintergrundsignal.

6. Verfahren nach Anspruch 5, wobei die Donatormoleküleinheit ein Fluorphor ist.

7. Verfahren nach Anspruch 5, wobei sich die erste und zweite Temperatur in den Inkubationsschritten um 5-30 °C unterscheiden.

8. Verfahren nach Anspruch 7, wobei sich die erste und zweite Temperatur in den Inkubationsschritten um 5-15 °C unterscheiden.

9. Verfahren nach Anspruch 5 oder 7, wobei Schritt e) das Messen im Zeitverlauf eines beliebigen des zweiten Signals, das möglicherweise während Schritt d) erzeugt worden ist, umfasst, wodurch eine Rate der Veränderung des in Schritt d) erzeugten Signals ermittelt wird.

10. Verfahren nach Anspruch 5, wobei Schritt c) das Identifizieren der Größenordnung des ersten Signals, das möglicherweise während Schritt b) erzeugt worden ist, umfasst und
wobei Schritt f) weiter das Bestimmen, dass die erste Zielnukleinsäure in der Probe vorhanden ist, wenn die identifizierte Größenordnung einen Größenordnungsschwellenwert zum Nachweis der ersten Zielnukleinsäure überschreitet, umfasst; oder
wobei Schritt c) das Identifizieren der Größenordnung des ersten Signals, das möglicherweise während Schritt b) erzeugt worden ist, umfasst und
wobei Schritt f) weiter das Bestimmen, dass die erste Zielnukleinsäure in der Probe nicht vorhanden ist, wenn die identifizierte Größenordnung einen Größenordnungsschwellenwert zum Nachweis der ersten Zielnukleinsäure nicht überschreitet, umfasst; oder
wobei Schritt e) das Messen im Zeitverlauf eines beliebigen des zweiten Signals, das möglicherweise während Schritt d) erzeugt worden ist, umfasst, wodurch eine Rate der Veränderung des in Schritt d) erzeugten Signals ermittelt wird, und
wobei Schritt f) weiter das Bestimmen, dass die zweite Zielnukleinsäure in der Probe vorhanden ist, wenn die Veränderungsrate einen Geschwindigkeitsschwellenwert zum Nachweis der zweiten Zielnukleinsäure überschreitet, umfasst; oder
wobei Schritt e) das Messen im Zeitverlauf eines beliebigen des zweiten Signals, das möglicherweise während Schritt d) erzeugt worden ist, umfasst, wodurch eine Rate der Veränderung des in Schritt d) erzeugten Signals ermittelt wird, und wobei Schritt f) weiter das Bestimmen, dass die zweite Zielnukleinsäure in der Probe nicht vorhanden ist, wenn die Veränderungsrate einen Geschwindigkeitsschwellenwert zum Nachweis der zweiten Zielnukleinsäure nicht überschreitet, umfasst.

11. Verfahren nach Anspruch 9, wobei die Veränderungsrate eine zeitabhängige Veränderungsrate ist; oder
wobei die Veränderungsrate eine zyklusabhängige Veränderungsrate ist; oder
wobei der Schwellenwert zum Nachweis des zweiten Ziels eine Schwellenwertgeschwindigkeit ist.

12. Verfahren nach einem der Ansprüche 5 bis 11, wobei die erste Temperatur von Schritt b) größer ist als die zweite Temperatur von Schritt d); und/oder
wobei der Schwellenwert zum Nachweis der ersten Zielnukleinsäure in Schritt f) eine Schwellenwertgrößenordnung ist; und/oder
wobei die Schritte b)-e) vor der Durchführung von Schritt f) eine Vielzahl von Malen nacheinander wiederholt werden.

## Revendications

1. Procédé pour déterminer lesquels parmi une pluralité d'acides nucléiques cibles différents sont présents dans un échantillon à tester, le procédé comprenant les étapes consistant à :
(a) dans un mélange réactionnel contenant des acides nucléiques provenant de l'échantillon à tester, permettre en série, dans l'ordre, à des première et seconde réactions secondaires d'un essai de clivage invasif multiplex d'avoir lieu, chacune des réactions secondaires étant active dans une condition de température différente, et chaque réaction secondaire étant spécifique d'un seul des acides nucléiques cibles ;
(b) surveiller le mélange réactionnel pour la production d'un signal fluorescent uniquement lorsque la seconde des deux réactions secondaires a lieu à la plus basse des deux conditions de température, chacune des différentes conditions de température étant permissive pour une seule des réactions secondaires, et chacune des réactions secondaires produisant le même signal fluorescent ; et
(c) déterminer lequel des acides nucléiques cibles est présent dans l'échantillon à tester en comparant une intensité du signal fluorescent produit dans le mélange réactionnel au cours de l'étape (b) à une valeur seuil ou un critère seuil.

2. Procédé selon la revendication 1, dans lequel l'échantillon à tester comprend des produits d'une réaction d'amplification d'acide nucléique, et dans lequel chacun de la pluralité d'acides nucléiques cibles est un produit de la réaction d'amplification d'acide nucléique ; ou
dans lequel l'échantillon à tester comprend des produits d'une réaction d'amplification d'acide nucléique multiplex, et dans lequel chacun de la pluralité d'acides nucléiques cibles est un produit de la réaction d'amplification d'acide nucléique multiplex.

3. Procédé selon l'une quelconque des revendications précédentes, dans lequel le mélange réactionnel comprend une enzyme ADN polymérase thermostable ; et/ou
dans lequel chacune des différentes conditions de température diffère des autres de 5 °C à 30 °C ; de préférence,
dans lequel chacune des différentes conditions de température diffère des autres de 5 °C à 15 °C.

4. Procédé selon l'une quelconque des revendications précédentes, dans lequel l'étape (c) consiste à déterminer lequel des acides nucléiques cibles est présent dans l'échantillon à tester sur la base de la quantité du signal fluorescent produit dans le mélange réactionnel au cours de l'étape (b) ; ou
dans lequel l'étape (c) consiste à déterminer lequel des acides nucléiques cibles est présent dans l'échantillon à tester sur la base du taux de production du signal fluorescent dans le mélange réactionnel au cours de l'étape (b).

5. Procédé pour déterminer si un échantillon à tester contient un ou les deux parmi un premier acide nucléique cible et un second acide nucléique cible, le procédé comprenant les étapes consistant à :
(a) préparer un mélange réactionnel en combinant chacun parmi,
(i) l'échantillon à tester,
(ii) un premier ensemble d'oligonucléotides pour effectuer un premier essai de clivage invasif spécifique pour la détection du premier acide nucléique cible, le premier essai de clivage invasif comprenant une réaction primaire et une réaction secondaire, et le premier ensemble d'oligonucléotides comprenant,
une première sonde invasive spécifique du premier acide nucléique cible,
une première sonde primaire spécifique du premier acide nucléique cible, et
une première cassette FRET,
(iii) un second ensemble d'oligonucléotides pour effectuer un second essai de clivage invasif spécifique pour la détection du second acide nucléique cible, le second essai de clivage invasif comprenant une réaction primaire et une réaction secondaire, le second ensemble d'oligonucléotides comprenant,
une seconde sonde invasive spécifique du second acide nucléique cible,
une seconde sonde primaire spécifique du second acide nucléique cible, et
une seconde cassette FRET,
dans lequel chacune des première et seconde cassettes FRET comprend un fragment donneur et un fragment accepteur en relation de transfert d'énergie de sorte qu'une émission provenant du fragment donneur est désactivée quand le fragment donneur et le fragment accepteur sont tous deux liés à la même cassette FRET, et
dans lequel le fragment donneur de la première cassette FRET est identique au fragment donneur de la seconde cassette FRET, et
(iv) une enzyme endonucléase à rabat (FEN) ;
(b) incuber le mélange réactionnel pendant une première période de temps à une première température, la première température étant permissive pour la réaction primaire des premier et second essais de clivage invasif, et pour la réaction primaire du premier essai de clivage invasif, selon lequel un premier signal associé au fragment donneur de la première cassette FRET est généré si l'échantillon à tester inclut le premier acide nucléique cible ;
(c) mesurer le premier signal quel qu'il soit qui peut avoir été généré au cours de l'étape (b) en tant qu'indication de la présence du premier acide nucléique cible dans l'échantillon à tester ;
(d) incuber le mélange réactionnel pendant une seconde période de temps à une seconde température, la seconde température étant permissive pour la réaction secondaire du second essai de clivage invasif, selon lequel un second signal associé au fragment donneur de la seconde cassette FRET est généré si l'échantillon à tester inclut le second acide nucléique cible ;
(e) mesurer le second signal quel qu'il soit qui peut avoir été généré au cours de l'étape (d) en tant qu'indication de la présence du second acide nucléique cible dans l'échantillon à tester ; et
(f) déterminer à partir du premier signal mesuré dans l'étape (c) si l'échantillon contient le premier acide nucléique cible, et à partir du second signal mesuré dans l'étape (e) si l'échantillon contient le second acide nucléique cible,
dans lequel la réaction secondaire du premier essai de clivage invasif ne génère sensiblement aucun signal supérieur à un signal de fond à la seconde température dans l'étape (d), et
dans lequel la réaction secondaire du second essai de clivage invasif ne génère sensiblement aucun signal supérieur à un signal de fond à la première température dans l'étape (b).

6. Procédé selon la revendication 5, dans lequel le fragment donneur est un fluorophore.

7. Procédé selon la revendication 5, dans lequel les première et seconde températures dans les étapes d'incubation diffèrent de 5 à 30 °C.

8. Procédé selon la revendication 7, dans lequel les première et seconde températures dans les étapes d'incubation diffèrent de 5 à 15 °C.

9. Procédé selon la revendication 5 ou 7, dans lequel l'étape (e) comprend la mesure au cours du temps du second signal quel qu'il soit qui peut avoir été généré au cours de l'étape (d), selon lequel une taux de changement du signal généré dans l'étape (d) est établi.

10. Procédé selon la revendication 5, dans lequel l'étape (c) comprend l'identification de l'intensité du premier signal qui peut avoir été généré au cours de l'étape (b), et
dans lequel l'étape (f) comprend en outre la détermination que le premier acide nucléique cible est présent dans l'échantillon si l'intensité identifiée dépasse un seuil d'intensité pour détecter le premier acide nucléique cible ; ou
dans lequel l'étape (c) comprend l'identification de l'intensité du premier signal qui peut avoir été généré au cours de l'étape (b), et
dans lequel l'étape (f) comprend en outre la détermination que le premier acide nucléique cible n'est pas présent dans l'échantillon si l'intensité identifiée ne dépasse pas un seuil d'intensité pour détecter le premier acide nucléique cible ; ou
dans lequel l'étape (e) comprend la mesure au cours du temps du second signal quel qu'il soit qui peut avoir été généré au cours de l'étape (d),
selon lequel un taux de changement du signal généré dans l'étape (d) est établi, et
dans lequel l'étape (f) comprend en outre la détermination que le second acide nucléique cible est présent dans l'échantillon si le taux de changement dépasse un seuil de vitesse pour détecter le second acide nucléique cible ; ou
dans lequel l'étape (e) comprend la mesure au cours du temps du second signal quel qu'il soit qui peut avoir été généré au cours de l'étape (d), selon lequel un taux de changement du signal généré dans l'étape (d) est établi, et dans lequel l'étape (f) comprend en outre la détermination que le second acide nucléique cible n'est pas présent dans l'échantillon si le taux de changement ne dépasse pas un seuil de vitesse pour détecter le second acide nucléique cible.

11. Procédé selon la revendication 9, dans lequel le taux de changement est un taux de changement dépendant du temps ; ou
dans lequel le taux de changement est un taux de changement dépendant du cycle ; ou
dans lequel le seuil pour détecter la seconde cible est une vitesse seuil.

12. Procédé selon l'une quelconque des revendications 5 à 11, dans lequel la première température de l'étape (b) est supérieure à la seconde température de l'étape (d) ; et/ou
dans lequel le seuil pour détecter le premier acide nucléique cible dans l'étape (f) est une intensité seuil ; et/ou
dans lequel les étapes (b) à (e) sont répétées en séquence plusieurs fois avant de mettre en œuvre l'étape (f).
